(19) Europäisches Patentamt
European Patent Office
Office européen des brevets

(11) EP 3 912 644 A1

(12) EUROPEAN PATENT APPLICATION
published in accordance with Art. 153(4) EPC

(43) Date of publication:
**24.11.2021 Bulletin 2021/47**

(21) Application number: **20741062.2**

(22) Date of filing: **17.01.2020**

(51) Int Cl.:
***A61K 48/00*** (2006.01) ***A61P 17/00*** (2006.01)
***A61P 17/02*** (2006.01) ***C12N 5/0775*** (2010.01)
***C12N 5/22*** (2006.01) ***C12N 15/12*** (2006.01)
***A61K 38/39*** (2006.01) ***A61K 35/28*** (2015.01)
***A61K 35/32*** (2015.01)

(86) International application number:
**PCT/JP2020/001433**

(87) International publication number:
**WO 2020/149395 (23.07.2020 Gazette 2020/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.01.2019 JP 2019007201**

(71) Applicant: **Osaka University**
**Suita-shi, Osaka 565-0871 (JP)**

(72) Inventors:
- **TAMAI, Katsuto**
**Suita-shi, Osaka 565-0871 (JP)**
- **KIKUCHI, Yasushi**
**Suita-shi, Osaka 565-0871 (JP)**
- **TAMAKOSHI, Tomoki**
**Suita-shi, Osaka 565-0871 (JP)**

(74) Representative: **Moré, Solveig Helga et al**
**Kroher Strobel**
**Rechts- und Patentanwälte PartmbB**
**Bavariaring 20**
**80336 München (DE)**

# (54) THERAPEUTIC AGENT FOR DYSTROPHIC EPIDERMOLYSIS BULLOSA

(57) The present disclosure relates to a composition for use in the treatment of dystrophic epidermolysis bullosa, comprising a cell obtained from a patient with dystrophic epidermolysis bullosa, wherein the cell is a mesenchymal stem cell and genetically modified to produce type VII collagen. The present disclosure also relates to a composition for use in the treatment of dystrophic epidermolysis bullosa, comprising a cell that produces type VII collagen, wherein the composition is to be administered into a blister.

Fig. 2

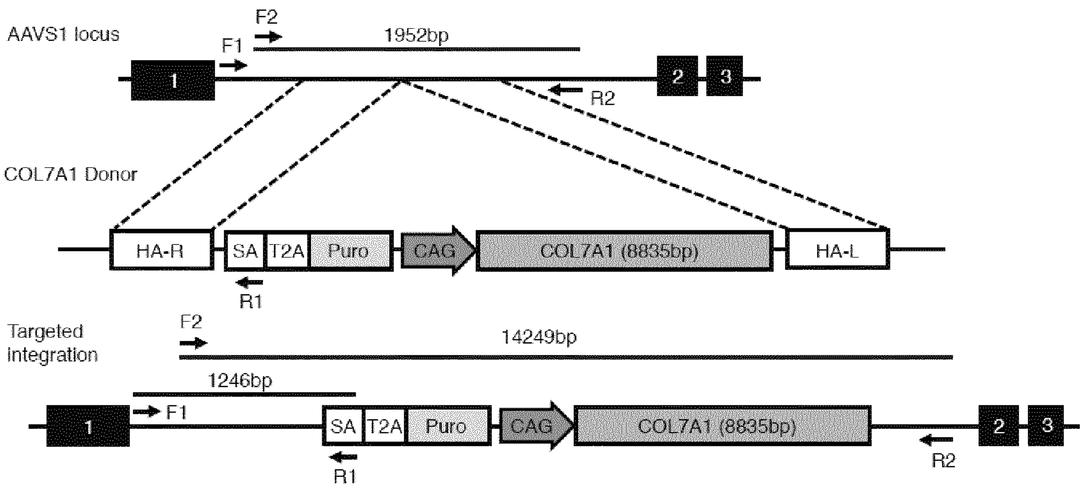


EP 3 912 644 A1

## Description

TECHNICAL FIELD

The present application claims priority with respect to Japanese Patent Application No. 2019-007201, which is incorporated herein by reference in its entirety.

The present disclosure relates to compositions for use in treating dystrophic epidermolysis bullosa.

BACKGROUND

Epidermolysis bullosa is a disease in which adhesive structural molecules responsible for adhesion of the skin tissue are lost or disappeared, and then the epidermis peels off from the dermis and blisters or skin ulcers occur when force is applied to the skin. The disease includes simple epidermolysis bullosa, in which the epidermis is torn to form blisters, junctional epidermolysis bullosa, in which the epidermis is peeled from the basement membrane to form blisters, and dystrophic epidermolysis bullosa, in which the basement membrane is peeled from the dermis.

Dystrophic epidermolysis bullosa is the most common type of epidermolysis bullosa, accounting for about 50% of all epidermolysis bullosa. It is a hereditary disease caused by a mutation in the COL7A1 gene, which encodes type VII collagen. In the structure of the skin, the epidermal basal cells at the bottom of the epidermis are bound to a sheet-like structure called the basement membrane. Type VII collagen forms fibers called anchoring fibrils in the dermis and connects the basement membrane and the dermis. Therefore, if there is an abnormality in the type VII collagen gene, the adhesive function between the basement membrane and the dermis is impaired, resulting in dystrophic epidermolysis bullosa, in which blisters form between the basement membrane and the dermis. Among dystrophic epidermolysis bullosa, severe recessive dystrophic epidermolysis bullosa is a very serious hereditary bullous skin disease that has continued burn-like skin symptoms throughout the body immediately after birth, and cutaneous spinous cell carcinoma (scar cancer) occurs frequently from around 30 years old and leads to death.

There is currently no effective treatment for epidermolysis bullosa, and the development of gene therapy that radically suppresses blistering is required. As such gene therapy, a therapeutic technique is disclosed in which skin cells of a patient are collected, genetically engineered to produce type VII collagen, cultured to form a skin sheet, and transplanted to the patient (Patent Document 1). Also, it has been proposed to subject mesenchymal stem cells lacking the type VII collagen activity to genome editing, differentiate the mesenchymal stem cells capable of producing type VII collagen thus obtained into keratinocytes or fibroblasts, culture the cells to form a skin sheet, and use the skin sheet for treating a patient (see Patent Document 2).

CITATION LIST

PATENT DOCUMENTS

Patent Document 1: WO2017/120147
Patent Document 2: WO2018/154413

SUMMARY OF INVENTION

PROBLEM TO BE SOLVED

Manufacturing the skin sheet requires advanced process control and culture technology, and then involves high-difficulty and high-cost. Therapeutic agents that are easier to manufacture are required.

SOLUTION TO PROBLEM

In one aspect, the present disclosure relates to a composition for use in the treatment of dystrophic epidermolysis bullosa, comprising a cell obtained from a patient with dystrophic epidermolysis bullosa, wherein the cell is a mesenchymal stem cell and genetically modified to produce type VII collagen.

In another aspect, the present disclosure relates to a composition for use in the treatment of dystrophic epidermolysis bullosa, comprising a cell that produces type VII collagen, wherein the composition is to be administered into a blister.

EFFECT OF INVENTION

The present disclosure provides compositions for use in treating dystrophic epidermolysis bullosa.

BRIEF DESCRIPTION OF DRAWINGS

Fig. 1 shows the cleavage of genomic DNA by the designed sgRNAs (sgAAVS1-#1 to #3) and their cleavage efficiency.

Fig. 2 is an explanatory diagram of genome editing in which a COL7A1 gene is introduced into the AAVS1 region. HA-R and HA-L indicate portions having homologous sequences, SA indicates a splice acceptor sequence, T2A indicates a T2A sequence encoding a T2A peptide, Puro indicates a puromycin resistance gene, and CAG indicates a CAG promoter sequence. The length from F2 to R2 in the wild-type genome (top) is 1952 bp, and the length from F1 to R1 and that from F2 to R2 in the genome into which the COL7A1 gene was introduced (bottom) is 1246 bp and 14249 bp, respectively.

Fis. 3 shows the gene transfer efficiency by the CRISPR-Cas9 system and the cell viability after gene transfer in mesenchymal stem cells (MSCs). The dashed line indicates the number of genome-edited cells, and the column indicates the cell viability.

Fig. 4 shows the results of confirming the introduction of the COL7A1 gene by genome editing.

Fig. 5 shows the expression of type VII collagen in MSCs. The symbol "(-)" indicates a control without genome editing, and the symbol "COL7A1-Donor" indicates genetically modified MSCs in which a COL7A1 gene was introduced by genome editing. The photo on the left shows the results of immunostaining of the cells, and the graph on the right shows the results of Western blotting of the culture supernatant of the cells.

Fig. 6 is an explanatory diagram of the production of epidermolysis bullosa model mice. The photo on the right shows the formed blisters.

Fig. 7 shows a skin tomographic image of an epidermolysis bullosa model mouse to which genetically modified MSCs were injected by intradermal or intrablister injection. The upper photo shows a merged image of DAPI staining and immunostaining for type VII collagen, and the lower photo shows the results of immunostaining for type VII collagen. The arrows indicate the expression of type VII collagen.

Fig. 8 shows a skin tomographic image of an epidermolysis bullosa model mouse to which genetically modified MSCs were injected by subcutaneous injection.

Fig. 9 shows an electron microscopic image of the skin of an epidermolysis bullosa model mouse to which genetically modified MSCs were injected by intrablister injection. The arrows indicate anchoring fibrils.

DESCRIPTION OF EMBODIMENTS

Unless otherwise specified, the terms used in the present disclosure have meanings generally understood by those skilled in the art in the fields such as organic chemistry, medical science, pharmaceutical science, molecular biology, and microbiology. Definitions of some terms used in the present disclosure are provided below, and these definitions supersede the general understandings in the present disclosure.

Dystrophic epidermolysis bullosa is a hereditary disease caused by a mutation in the COL7A1 gene, which encodes type VII collagen, and is known to be characterized in that no type VII collagen is produced or type VII collagen with reduced function due to the mutation is produced. The type VII collagen forms fibers called anchoring fibrils in the dermis and connects the basement membrane and the dermis. The type VII collagen contains a first non-collagen region, a collagen region, and a second non-collagen region from the N-terminus, and forms a triple chain at the collagen region, which is characterized by a repeating sequence of glycine-X-Y. Two molecules bind to each other at the C-terminus and the N-terminus binds to the basement membrane. Examples of mutations include a mutation in which glycine in the collagen region is replaced by a different amino acid, a stop codon mutation that stops protein translation, and a splice site mutation. The mutation may be in one of the alleles or in both. Dystrophic epidermolysis bullosa includes dominant dystrophic epidermolysis bullosa and recessive dystrophic epidermolysis bullosa, and the recessive dystrophic epidermolysis bullosa include severe generalized recessive dystrophic epidermolysis bullosa and other generalized types with relatively mild symptoms. The dystrophic epidermolysis bullosa herein may be any type of dystrophic epidermolysis bullosa, and the causal mutation in the COL7A1 gene may be any mutation.

In the present disclosure, a cell that produces type VII collagen is used. As used herein, the term "cell that produces type VII collagen" means a cell that produces a functional type VII collagen (ie, a type VII collagen capable of forming anchoring fibrils). The cell that produces type VII collagen may be a cell that naturally produces type VII collagen or a cell that has been genetically modified to produce type VII collagen.

In the present disclosure, genetic modification of a cell means both modification of a gene in the genome of the cell and modification of the cell to express a gene from a nucleic acid construct outside the genome (such as a vector) . That is, the expression "genetically modifying a cell to produce type VII collagen" includes modifying a cell to express type VII collagen from a COL7A1 gene in the genome, and modifying a cell to express type VII collagen from a COL7A1 gene in a nucleic acid construct outside the genome. Also, "a cell genetically modified to produce type VII collagen" includes a cell that expresses type VII collagen from a COL7A1 gene in the genome and a cell that expresses type VII collagen from a COL7A1 gene in a nucleic acid construct outside the genome.

Genetic modification of a cell can be carried out by introducing a COL7A1 gene or by correcting a mutation in the COL7A1 gene in the genome. The introduction of a COL7A1 gene can be carried out either by introducing a COL7A1 gene into the genome of the cell or by placing a nucleic acid construct comprising a COL7A1 gene in the cell so that the COL7A1 gene is expressed from the nucleic acid construct outside the genome. When a COL7A1 gene is introduced into the genome of a cell, the COL7A1 gene may be introduced at a specific site or may be introduced at random. In an embodiment, the COL7A1 gene is introduced into the COL7A1 locus of the genome, or a safe harbor such as the AAVS1 region.

The cell may be a cell obtained from a patient with dystrophic epidermolysis bullosa to which the cell is to be administered (ie, an autologous cell), or a cell obtained from a subject other than the patient (ie, an allogeneic cell). Subjects other than the patient include healthy individuals, especially HLA-matched healthy individuals, or the patient's mother. In an embodiment, the cell is a cell obtained from a patient with dystrophic epidermolysis bullosa. The cell obtained from a patient with dystrophic epidermolysis bullosa includes a cell that does not produce type VII collagen and a cell that produces type VII collagen with reduced function due to a mutation, and the "cell obtained from a patient with dystrophic epidermolysis bullosa" as used herein may be any of them.

The cell may be any cell as long as it produces type VII collagen in the vicinity of the epidermal basement membrane when administered to a patient. The cell can be a cell derived from skin, bone marrow, or blood (eg, peripheral blood) . In an embodiment, the cell is a keratinocyte, skin fibroblast, or mesenchymal stem cell. In a different embodiment, the cell is an iPS cell induced from a cell obtained from a patient or a subject other than the patient or a cell induced from such an iPS cell. Thus, the cell may be a cell obtained from a patient or a subject other than the patient, or may be a cell induced from the obtained cell. When the cell is a genetically modified cell and also it is a cell induced from a cell obtained from a patient or a subject other than the patient, the genetic modification may have been carried out before or after the induction.

In the present disclosure, the term "cell" is used in the sense of including a cell after proliferation as needed. Proliferation of a cell can be carried out by culturing the cell. For example, "a cell obtained from a patient or a subject other than the patient" includes a cell collected from a patient or a subject other than the patient and then proliferated, and "a genetically modified cell" includes a cell that is proliferated from a cell obtained by genetic modification. When genetic modification is carried out, a cell may be prolifelated until the amount required for the genetic modification is obtained. Also, after genetic modification, the cell may be prolifelated until the amount required for treatment is obtained.

Keratinocytes and skin fibroblasts may be obtained by any method known in the art. For example, the epidermis and the dermis are separated by enzymatic treatment and/or mechanical treatment of the skin biopsy tissue, and each of the epidermis and the dermis thus separated is further subjected to enzymatic treatment. Keratinocytes can be obtained from the epidermis sample and skin fibroblasts can be obtained from the dermis sample.

In an embodiment, the cell is a mesenchymal stem cell. When administered to a patient, mesenchymal stem cells are considered to reside in a patient tissue longer than keratinocytes and skin fibroblasts. Also, while inflammatory reactions are expected when a genetically modified cell produces a protein that has not been produced in the patient so far, mesenchymal stem cells have an anti-inflammatory effect and thus would more advantageous than keratinocytes and skin fibroblasts.

A mesenchymal stem cell (also referred to herein as MSC) has adhesiveness to a solid phase (eg, a plastic culture vessel), and has both the self-renewal ability and the differentiation ability into mesenchymal tissues (such as bone, cartilage, fat, and muscle). In an embodiment, the mesenchymal stem cell is a cell capable of differentiating into at least one of an osteoblast, chondrocyte and adipocyte. In an embodiment, the mesenchymal stem cell is a cell capable of differentiating into an osteoblast, chondrocyte and adipocyte. When a cell population has the above-mentioned abilities, it is understood to include a mesenchymal stem cell. Mesenchymal stem cells can be obtained from bone marrow or other tissues (for example, blood, such as umbilical cord blood and peripheral blood, as well as skin, fat, and dental pulp). In an embodiment, the mesenchymal stem cell is a bone marrow-derived mesenchymal stem cell (also referred to herein as BM-MSC). The bone marrow-derived mesenchymal stem cell can be obtained from any site such as femur, vertebra, sternum, ilium, and tibia.

Mesenchymal stem cells may be obtained by any method known in the art. For example, methods based on adhesiveness, cell surface markers, and density difference can be mentioned. For example, cells obtained from bone marrow or other tissues containing mesenchymal stem cells are seeded on a plastic or glass culture vessel, and cells that adhere to the culture vessel and proliferate are collected. Alternatively, mesenchymal stem cells can also be obtained by cell sorting (such as FACS, MACS) using an antibody against a surface marker of mesenchymal stem cells. The surface marker of human mesenchymal stem cells may be one or more of the followings: PDGFRα positive, PDGFRβ positive, Lin negative, CD45 negative, CD44 positive, CD90 positive, CD29 positive, Flk-1 negative, CD105 positive, CD73 positive , CD90 positive, CD71 positive, Stro-1 positive, CD106 positive, CD166 positive, CD31 negative, CD271 positive, and CD11b negative.

iPS cells may be produced by any method known in the art. For example, iPS cells can be produced by introducing three types of transcription factors, OCT4, SOX2, and NANOG into somatic cells such as fibroblasts obtained from a patient or a subject other than the patient (Budniatzky and Gepstein, Stem Cells Transl Med. 3(4) :448-57, 2014 ; Barrett et al, Stem Cells Trans Med 3 : 1-6 sctm.2014-0121, 2014 ; Focosi et al., Blood Cancer Journal 4: e211, 2014).

As used herein, the term "cell" can mean a single cell or multiple cells, depending on the context. Further, the cell may be a cell population composed of one type of cell or a cell population including a plurality of types of cells.

As used herein, the term "COL7A1 gene" means a nucleic acid sequence encoding type VII collagen, and is used to include cDNA as well as a sequence containing one or more introns (for example, a genomic sequence or a minigene). The representative nucleic acid sequence of the human COL7A1 gene (cDNA) is shown in SEQ ID NO: 1, and the representative amino acid sequence of human type VII collagen is shown in SEQ ID NO: 2. The cDNA sequence of the COL7A1 gene is disclosed in GenBank: NM_000094.3, and the genome sequence is disclosed in GenBank: AC121252.4. The sequence of a COL7A1 gene is not limited to any specific sequence as long as it encodes a functional type VII collagen (ie, a type VII collagen capable of forming anchoring fibrils).

cDNA sequence of human COL7A1 gene (8835bp) (SEQ ID NO: 1)

ATGACGCTGCGGCTTCTGGTGGCCGCGCTCTGCGCCGGGATCCTGGCAGAGGCGCCCCG

AGTGCGAGCCCAGCACAGGGAGAGAGTGACCTGCACGCGCCTTTACGCCGCTGACATTG
TGTTCTTACTGGATGGCTCCTCATCCATTGGCCGCAGCAATTTCCGCGAGGTCCGCAGC
TTTCTCGAAGGGCTGGTGCTGCCTTTCTCTGGAGCAGCCAGTGCACAGGGTGTGCGCTT
TGCCACAGTGCAGTACAGCGATGATCCACGGACAGAGTTCGGCCTGGATGCACTTGGCT
CTGGGGGTGATGTGATCCGCGCCATCCGTGAGCTTAGCTACAAGGGGGGCAACACTCGC
ACAGGGGCTGCAATTCTCCATGTGGCTGACCATGTCTTCCTGCCCCAGCTGGCCCGACC
TGGTGTCCCCAAGGTCTGCATCCTGATCACAGACGGGAAGTCCCAGGACCTGGTGGACA
CAGCTGCCCAAAGGCTGAAGGGGCAGGGGGTCAAGCTATTTGCTGTGGGGATCAAGAAT
GCTGACCCTGAGGAGCTGAAGCGAGTTGCCTCACAGCCCACCAGTGACTTCTTCTTCTT
CGTCAATGACTTCAGCATCTTGAGGACACTACTGCCCCTCGTTTCCCGGAGAGTGTGCA
CGACTGCTGGTGGCGTGCCTGTGACCCGACCTCCGGATGACTCGACCTCTGCTCCACGA
GACCTGGTGCTGTCTGAGCCAAGCAGCCAATCCTTGAGAGTACAGTGGACAGCGGCCAG
TGGCCCTGTGACTGGCTACAAGGTCCAGTACACTCCTCTGACGGGGCTGGGACAGCCAC
TGCCGAGTGAGCGGCAGGAGGTGAACGTCCCAGCTGGTGAGACCAGTGTGCGGCTGCGG
GGTCTCCGGCCACTGACCGAGTACCAAGTGACTGTGATTGCCCTCTACGCCAACAGCAT
CGGGGAGGCTGTGAGCGGGACAGCTCGGACCACTGCCCTAGAAGGGCCGGAACTGACCA
TCCAGAATACCACAGCCCACAGCCTCCTGGTGGCCTGGCGGAGTGTGCCAGGTGCCACT
GGCTACCGTGTGACATGGCGGGTCCTCAGTGGTGGGCCCACACAGCAGCAGGAGCTGGG
CCCTGGGCAGGGTTCAGTGTTGCTGCGTGACTTGGAGCCTGGCACGGACTATGAGGTGA
CCGTGAGCACCCTATTTGGCCGCAGTGTGGGGCCCGCCACTTCCCTGATGGCTCGCACT
GACGCTTCTGTTGAGCAGACCCTGCGCCCGGTCATCCTGGGCCCCACATCCATCCTCCT
TTCCTGGAACTTGGTGCCTGAGGCCCGTGGCTACCGGTTGGAATGGCGGCGTGAGACTG
GCTTGGAGCCACCGCAGAAGGTGGTACTGCCCTCTGATGTGACCCGCTACCAGTTGGAT
GGGCTGCAGCCGGGCACTGAGTACCGCCTCACACTCTACACTCTGCTGGAGGGCCACGA
GGTGGCCACCCCTGCAACCGTGGTTCCCACTGGACCAGAGCTGCCTGTGAGCCCTGTAA
CAGACCTGCAAGCCACCGAGCTGCCCGGGCAGCGGGTGCGAGTGTCCTGGAGCCCAGTC
CCTGGTGCCACCCAGTACCGCATCATTGTGCGCAGCACCCAGGGGGTTGAGCGGACCCT
GGTGCTTCCTGGGAGTCAGACAGCATTCGACTTGGATGACGTTCAGGCTGGGCTTAGCT
ACACTGTGCGGGTGTCTGCTCGAGTGGGTCCCCGTGAGGGCAGTGCCAGTGTCCTCACT
GTCCGCCGGGAGCCGGAAACTCCACTTGCTGTTCCAGGGCTGCGGGTTGTGGTGTCAGA
TGCAACGCGAGTGAGGGTGGCCTGGGGACCCGTCCCTGGAGCCAGTGGATTTCGGATTA
GCTGGAGCACAGGCAGTGGTCCGGAGTCCAGCCAGACACTGCCCCCAGACTCTACTGCC
ACAGACATCACAGGGCTGCAGCCTGGAACCACCTACCAGGTGGCTGTGTCGGTACTGCG
AGGCAGAGAGGAGGGCCCTGCTGCAGTCATCGTGGCTCGAACGGACCCACTGGGCCCAG
TGAGGACGGTCCATGTGACTCAGGCCAGCAGCTCATCTGTCACCATTACCTGGACCAGG

GTTCCTGGCGCCACAGGATACAGGGTTTCCTGGCACTCAGCCCACGGCCCAGAGAAATC
CCAGTTGGTTTCTGGGGAGGCCACGGTGGCTGAGCTGGATGGACTGGAGCCAGATACTG
AGTATACGGTGCATGTGAGGGCCCATGTGGCTGGCGTGGATGGCCCCCTGCCTCTGTG
GTTGTGAGGACTGCCCCTGAGCCTGTGGGTCGTGTGTCGAGGCTGCAGATCCTCAATGC
TTCCAGCGACGTTCTACGGATCACCTGGGTAGGGGTCACTGGAGCCACAGCTTACAGAC
TGGCCTGGGGCCGGAGTGAAGGCGGCCCCATGAGGCACCAGATACTCCCAGGAAACACA
GACTCTGCAGAGATCCGGGGTCTCGAAGGTGGAGTCAGCTACTCAGTGCGAGTGACTGC
ACTTGTCGGGGACCGCGAGGGCACACCTGTCTCCATTGTTGTCACTACGCCGCCTGAGG
CTCCGCCAGCCCTGGGGACGCTTCACGTGGTGCAGCGCGGGGAGCACTCGCTGAGGCTG
CGCTGGGAGCCGGTGCCCAGAGCGCAGGGCTTCCTTCTGCACTGGCAACCTGAGGGTGG
CCAGGAACAGTCCCGGGTCCTGGGGCCCGAGCTCAGCAGCTATCACCTGGACGGGCTGG
AGCCAGCGACACAGTACCGCGTGAGGCTGAGTGTCCTAGGGCCAGCTGGAGAAGGGCCC
TCTGCAGAGGTGACTGCGCGCACTGAGTCACCTCGTGTTCCAAGCATTGAACTACGTGT
GGTGGACACCTCGATCGACTCGGTGACTTTGGCCTGGACTCCAGTGTCCAGGGCATCCA
GCTACATCCTATCCTGGCGGCCACTCAGAGGCCCTGGCCAGGAAGTGCCTGGGTCCCCG
CAGACACTTCCAGGGATCTCAAGCTCCCAGCGGGTGACAGGGCTAGAGCCTGGCGTCTC
TTACATCTTCTCCCTGACGCCTGTCCTGGATGGTGTGCGGGGTCCTGAGGCATCTGTCA
CACAGACGCCAGTGTGCCCCCGTGGCCTGGCGGATGTGGTGTTCCTACCACATGCCACT
CAAGACAATGCTCACCGTGCGGAGGCTACGAGGAGGGTCCTGGAGCGTCTGGTGTTGGC
ACTTGGGCCTCTTGGGCCACAGGCAGTTCAGGTTGGCCTGCTGTCTTACAGTCATCGGC
CCTCCCCACTGTTCCCACTGAATGGCTCCCATGACCTTGGCATTATCTTGCAAAGGATC
CGTGACATGCCCTACATGGACCCAAGTGGGAACAACCTGGGCACAGCCGTGGTCACAGC
TCACAGATACATGTTGGCACCAGATGCTCCTGGGCGCCGCCAGCACGTACCAGGGGTGA
TGGTTCTGCTAGTGGATGAACCCTTGAGAGGTGACATATTCAGCCCCATCCGTGAGGCC
CAGGCTTCTGGGCTTAATGTGGTGATGTTGGGAATGGCTGGAGCGGACCCAGAGCAGCT
GCGTCGCTTGGCGCCGGGTATGGACTCTGTCCAGACCTTCTTCGCCGTGGATGATGGGC
CAAGCCTGGACCAGGCAGTCAGTGGTCTGGCCACAGCCCTGTGTCAGGCATCCTTCACT
ACTCAGCCCCGGCCAGAGCCCTGCCCAGTGTATTGTCCAAAGGGCCAGAAGGGGGAACC
TGGAGAGATGGGCCTGAGAGGACAAGTTGGGCCTCCTGGCGACCCTGGCCTCCCGGGCA
GGACCGGTGCTCCCGGCCCCCAGGGGCCCCCTGGAAGTGCCACTGCCAAGGGCGAGAGG
GGCTTCCCTGGAGCAGATGGGCGTCCAGGCAGCCCTGGCCGCGCCGGGAATCCTGGGAC
CCCTGGAGCCCCTGGCCTAAAGGGCTCTCCAGGGTTGCCTGGCCCTCGTGGGGACCCGG
GAGAGCGAGGACCTCGAGGCCCAAAGGGGGAGCCGGGGGCTCCCGGACAAGTCATCGGA
GGTGAAGGACCTGGGCTTCCTGGGCGGAAAGGGGACCCTGGACCATCGGGCCCCCCTGG
ACCTCGTGGACCACTGGGGGACCCAGGACCCCGTGGCCCCCCAGGGCTTCCTGGAACAG

CCATGAAGGGTGACAAAGGCGATCGTGGGGAGCGGGGTCCCCCTGGACCAGGTGAAGGT
GGCATTGCTCCTGGGGAGCCTGGGCTGCCGGGTCTTCCCGGAAGCCCTGGACCCCAAGG
CCCCGTTGGCCCCCCTGGAAAGAAAGGAGAAAAAGGTGACTCTGAGGATGGAGCTCCAG
GCCTCCCAGGACAACCTGGGTCTCCGGGTGAGCAGGGCCCACGGGGACCTCCTGGAGCT
ATTGGCCCCAAAGGTGACCGGGGCTTTCCAGGGCCCCTGGGTGAGGCTGGAGAGAAGGG
CGAACGTGGACCCCCAGGCCCAGCGGGATCCCGGGGGCTGCCAGGGGTTGCTGGACGTC
CTGGAGCCAAGGGTCCTGAAGGGCCACCAGGACCCACTGGCCGCCAAGGAGAGAAGGGG
GAGCCTGGTCGCCCTGGGGACCCTGCAGTGGTGGGACCTGCTGTTGCTGGACCCAAAGG
AGAAAAGGGAGATGTGGGGCCCGCTGGGCCCAGAGGAGCTACCGGAGTCCAAGGGGAAC
GGGGCCCACCCGGCTTGGTTCTTCCTGGAGACCCTGGCCCCAAGGGAGACCCTGGAGAC
CGGGGTCCCATTGGCCTTACTGGCAGAGCAGGACCCCCAGGTGACTCAGGGCCTCCTGG
AGAGAAGGGAGACCCTGGGCGGCCTGGCCCCCCAGGACCTGTTGGCCCCCGAGGACGAG
ATGGTGAAGTTGGAGAGAAAGGTGACGAGGGTCCTCCGGGTGACCCGGGTTTGCCTGGA
AAAGCAGGCGAGCGTGGCCTTCGGGGGGCACCTGGAGTTCGGGGGCCTGTGGGTGAAAA
GGGAGACCAGGGAGATCCTGGAGAGGATGGACGAAATGGCAGCCCTGGATCATCTGGAC
CCAAGGGTGACCGTGGGGAGCCGGGTCCCCCAGGACCCCCGGGACGGCTGGTAGACACA
GGACCTGGAGCCAGAGAGAAGGGAGAGCCTGGGGACCGCGGACAAGAGGGTCCTCGAGG
GCCCAAGGGTGATCCTGGCCTCCCTGGAGCCCCTGGGGAAAGGGGCATTGAAGGGTTTC
GGGGACCCCCAGGCCCACAGGGGGACCCAGGTGTCCGAGGCCCAGCAGGAGAAAAGGGT
GACCGGGGTCCCCCTGGGCTGGATGGCCGGAGCGGACTGGATGGGAAACCAGGAGCCGC
TGGGCCCTCTGGGCCGAATGGTGCTGCAGGCAAAGCTGGGGACCCAGGGAGAGACGGGC
TTCCAGGCCTCCGTGGAGAACAGGGCCTCCCTGGCCCCTCTGGTCCCCCTGGATTACCG
GGAAAGCCAGGCGAGGATGGCAAACCTGGCCTGAATGGAAAAAACGGAGAACCTGGGGA
CCCTGGAGAAGACGGGAGGAAGGGAGAGAAAGGAGATTCAGGCGCCTCTGGGAGAGAAG
GTCGTGATGGCCCCAAGGGTGAGCGTGGAGCTCCTGGTATCCTTGGACCCCAGGGGCCT
CCAGGCCTCCCAGGGCCAGTGGGCCCTCCTGGCCAGGGTTTTCCTGGTGTCCCAGGAGG
CACGGGCCCCAAGGGTGACCGTGGGGAGACTGGATCCAAAGGGGAGCAGGGCCTCCCTG
GAGAGCGTGGCCTGCGAGGAGAGCCTGGAAGTGTGCCGAATGTGGATCGGTTGCTGGAA
ACTGCTGGCATCAAGGCATCTGCCCTGCGGGAGATCGTGGAGACCTGGGATGAGAGCTC
TGGTAGCTTCCTGCCTGTGCCCGAACGGCGTCGAGGCCCCAAGGGGGACTCAGGCGAAC
AGGGCCCCCCAGGCAAGGAGGGCCCCATCGGCTTTCCTGGAGAACGCGGGCTGAAGGGC
GACCGTGGAGACCCTGGCCCTCAGGGGCCACCTGGTCTGGCCCTTGGGGAGAGGGGCCC
CCCCGGGCCTTCCGGCCTTGCCGGGGAGCCTGGAAAGCCTGGTATTCCCGGGCTCCCAG
GCAGGGCTGGGGGTGTGGGAGAGGCAGGAAGGCCAGGAGAGAGGGGAGAACGGGGAGAG
AAAGGAGAACGTGGAGAACAGGGCAGAGATGGCCCTCCTGGACTCCCTGGAACCCCTGG

GCCCCCCGGACCCCCTGGCCCCAAGGTGTCTGTGGATGAGCCAGGTCCTGGACTCTCTG
GAGAACAGGGACCCCCTGGACTCAAGGGTGCTAAGGGGGAGCCGGGCAGCAATGGTGAC
CAAGGTCCCAAAGGAGACAGGGGTGTGCCAGGCATCAAAGGAGACCGGGGAGAGCCTGG
ACCGAGGGGTCAGGACGGCAACCCGGGTCTACCAGGAGAGCGTGGTATGGCTGGGCCTG
AAGGGAAGCCGGGTCTGCAGGGTCCAAGAGGCCCCCCTGGCCCAGTGGGTGGTCATGGA
GACCCTGGACCACCTGGTGCCCCGGGTCTTGCTGGCCCTGCAGGACCCCAAGGACCTTC
TGGCCTGAAGGGGGAGCCTGGAGAGACAGGACCTCCAGGACGGGGCCTGACTGGACCTA
CTGGAGCTGTGGGACTTCCTGGACCCCCCGGCCCTTCAGGCCTTGTGGGTCCACAGGGG
TCTCCAGGTTTGCCTGGACAAGTGGGGGAGACAGGGAAGCCGGGAGCCCCAGGTCGAGA
TGGTGCCAGTGGAAAAGATGGAGACAGAGGGAGCCCTGGTGTGCCAGGGTCACCAGGTC
TGCCTGGCCCTGTCGGACCTAAAGGAGAACCTGGCCCCACGGGGGCCCCTGGACAGGCT
GTGGTCGGGCTCCCTGGAGCAAAGGGAGAGAAGGGAGCCCCTGGAGGCCTTGCTGGAGA
CCTGGTGGGTGAGCCGGGAGCCAAAGGTGACCGAGGACTGCCAGGGCCGCGAGGCGAGA
AGGGTGAAGCTGGCCGTGCAGGGGAGCCCGGAGACCCTGGGGAAGATGGTCAGAAAGGG
GCTCCAGGACCCAAAGGTTTCAAGGGTGACCCAGGAGTCGGGGTCCCGGGCTCCCCTGG
GCCTCCTGGCCCTCCAGGTGTGAAGGGAGATCTGGGCCTCCCTGGCCTGCCCGGTGCTC
CTGGTGTTGTTGGGTTCCCGGGTCAGACAGGCCCTCGAGGAGAGATGGGTCAGCCAGGC
CCTAGTGGAGAGCGGGGTCTGGCAGGCCCCCCAGGGAGAGAAGGAATCCCAGGACCCCT
GGGGCCACCTGGACCACCGGGGTCAGTGGGACCACCTGGGGCCTCTGGACTCAAAGGAG
ACAAGGGAGACCCTGGAGTAGGGCTGCCTGGGCCCCGAGGCGAGCGTGGGGAGCCAGGC
ATCCGGGGTGAAGATGGCCGCCCCGGCCAGGAGGGACCCCGAGGACTCACGGGGCCCCC
TGGCAGCAGGGGAGAGCGTGGGGAGAAGGGTGATGTTGGGAGTGCAGGACTAAAGGGTG
ACAAGGGAGACTCAGCTGTGATCCTGGGGCCTCCAGGCCCACGGGGTGCCAAGGGGGAC
ATGGGTGAACGAGGGCCTCGGGGCTTGGATGGTGACAAAGGACCTCGGGGAGACAATGG
GGACCCTGGTGACAAGGGCAGCAAGGGAGAGCCTGGTGACAAGGGCTCAGCCGGGTTGC
CAGGACTGCGTGGACTCCTGGGACCCCAGGGTCAACCTGGTGCAGCAGGGATCCCTGGT
GACCCGGGATCCCCAGGAAAGGATGGAGTGCCTGGTATCCGAGGAGAAAAAGGAGATGT
TGGCTTCATGGGTCCCCGGGGCCTCAAGGGTGAACGGGGAGTGAAGGGAGCCTGTGGCC
TTGATGGAGAGAAGGGAGACAAGGGAGAAGCTGGTCCCCCAGGCCGCCCCGGGCTGGCA
GGACACAAAGGAGAGATGGGGGAGCCTGGTGTGCCGGGCCAGTCGGGGGCCCCTGGCAA
GGAGGGCCTGATCGGTCCCAAGGGTGACCGAGGCTTTGACGGGCAGCCAGGCCCCAAGG
GTGACCAGGGCGAGAAAGGGGAGCGGGGAACCCCAGGAATTGGGGGCTTCCCAGGCCCC
AGTGGAAATGATGGCTCTGCTGGTCCCCCAGGGCCACCTGGCAGTGTTGGTCCCAGAGG
CCCCGAAGGACTTCAGGGCCAGAAGGGTGAGCGAGGTCCCCCCGGAGAGAGAGTGGTGG
GGGCTCCTGGGGTCCCTGGAGCTCCTGGCGAGAGAGGGGAGCAGGGGCGGCCAGGGCCT

GCCGGTCCTCGAGGCGAGAAGGGAGAAGCTGCACTGACGGAGGATGACATCCGGGGCTT
TGTGCGCCAAGAGATGAGTCAGCACTGTGCCTGCCAGGGCCAGTTCATCGCATCTGGAT
CACGACCCCTCCCTAGTTATGCTGCAGACACTGCCGGCTCCCAGCTCCATGCTGTGCCT
GTGCTCCGCGTCTCTCATGCAGAGGAGGAAGAGCGGGTACCCCCTGAGGATGATGAGTA
CTCTGAATACTCCGAGTATTCTGTGGAGGAGTACCAGGACCCTGAAGCTCCTTGGGATA
GTGATGACCCCTGTTCCCTGCCACTGGATGAGGGCTCCTGCACTGCCTACACCCTGCGC
TGGTACCATCGGGCTGTGACAGGCAGCACAGAGGCCTGTCACCCTTTTGTCTATGGTGG
CTGTGGAGGGAATGCCAACCGTTTTGGGACCCGTGAGGCCTGCGAGCGCCGCTGCCCAC
CCCGGGTGGTCCAGAGCCAGGGGACAGGTACTGCCCAGGACTGA

Amino acid sequence of human type VII collagen (2944 AA) (SEQ ID NO: 2)

MTLRLLVAALCAGILAEAPRVRAQHRERVTCTRLYAADIVFLLDGSSSIGRSNFREVRS
FLEGLVLPFSGAASAQGVRFATVQYSDDPRTEFGLDALGSGGDVIRAIRELSYKGGNTR
TGAAILHVADHVFLPQLARPGVPKVCILITDGKSQDLVDTAAQRLKGQGVKLFAVGIKN
ADPEELKRVASQPTSDFFFFVNDFSILRTLLPLVSRRVCTTAGGVPVTRPPDDSTSAPR
DLVLSEPSSQSLRVQWTAASGPVTGYKVQYTPLTGLGQPLPSERQEVNVPAGETSVRLR
GLRPLTEYQVTVIALYANSIGEAVSGTARTTALEGPELTIQNTTAHSLLVAWRSVPGAT
GYRVTWRVLSGGPTQQQELGPGQGSVLLRDLEPGTDYEVTVSTLFGRSVGPATSLMART
DASVEQTLRPVILGPTSILLSWNLVPEARGYRLEWRRETGLEPPQKVVLPSDVTRYQLD
GLQPGTEYRLTLYTLLEGHEVATPATVVPTGPELPVSPVTDLQATELPGQRVRVSWSPV
PGATQYRIIVRSTQGVERTLVLPGSQTAFDLDDVQAGLSYTVRVSARVGPREGSASVLT
VRREPETPLAVPGLRVVVSDATRVRVAWGPVPGASGFRISWSTGSGPESSQTLPPDSTA
TDITGLQPGTTYQVAVSVLRGREEGPAAVIVARTDPLGPVRTVHVTQASSSSVTITWTR
VPGATGYRVSWHSAHGPEKSQLVSGEATVAELDGLEPDTEYTVHVRAHVAGVDGPPASV
VVRTAPEPVGRVSRLQILNASSDVLRITWVGVTGATAYRLAWGRSEGGPMRHQILPGNT
DSAEIRGLEGGVSYSVRVTALVGDREGTPVSIVVTTPPEAPPALGTLHVVQRGEHSLRL
RWEPVPRAQGFLLHWQPEGGQEQSRVLGPELSSYHLDGLEPATQYRVRLSVLGPAGEGP
SAEVTARTESPRVPSIELRVVDTSIDSVTLAWTPVSRASSYILSWRPLRGPGQEVPGSP
QTLPGISSSQRVTGLEPGVSYIFSLTPVLDGVRGPEASVTQTPVCPRGLADVVFLPHAT
QDNAHRAEATRRVLERLVLALGPLGPQAVQVGLLSYSHRPSPLFPLNGSHDLGIILQRI
RDMPYMDPSGNNLGTAVVTAHRYMLAPDAPGRRQHVPGVMVLLVDEPLRGDIFSPIREA
QASGLNVVMLGMAGADPEQLRRLAPGMDSVQTFFAVDDGPSLDQAVSGLATALCQASFT
TQPRPEPCPVYCPKGQKGEPGEMGLRGQVGPPGDPGLPGRTGAPGPQGPPGSATAKGER
GFPGADGRPGSPGRAGNPGTPGAPGLKGSPGLPGPRGDPGERGPRGPKGEPGAPGQVIG

GEGPGLPGRKGDPGPSGPPGPRGPLGDPGPRGPPGLPGTAMKGDKGDRGERGPPGPGEG
GIAPGEPGLPGLPGSPGPQGPVGPPGKKGEKGDSEDGAPGLPGQPGSPGEQGPRGPPGA
IGPKGDRGFPGPLGEAGEKGERGPPGPAGSRGLPGVAGRPGAKGPEGPPGPTGRQGEKG
EPGRPGDPAVVGPAVAGPKGEKGDVGPAGPRGATGVQGERGPPGLVLPGDPGPKGDPGD
RGPIGLTGRAGPPGDSGPPGEKGDPGRPGPPGPVGPRGRDGEVGEKGDEGPPGDPGLPG
KAGERGLRGAPGVRGPVGEKGDQGDPGEDGRNGSPGSSGPKGDRGEPGPPGPPGRLVDT
GPGAREKGEPGDRGQEGPRGPKGDPGLPGAPGERGIEGFRGPPGPQGDPGVRGPAGEKG
DRGPPGLDGRSGLDGKPGAAGPSGPNGAAGKAGDPGRDGLPGLRGEQGLPGPSGPPGLP
GKPGEDGKPGLNGKNGEPGDPGEDGRKGEKGDSGASGREGRDGPKGERGAPGILGPQGP
PGLPGPVGPPGQGFPGVPGGTGPKGDRGETGSKGEQGLPGERGLRGEPGSVPNVDRLLE
TAGIKASALREIVETWDESSGSFLPVPERRRGPKGDSGEQGPPGKEGPIGFPGERGLKG
DRGDPGPQGPPGLALGERGPPGPSGLAGEPGKPGIPGLPGRAGGVGEAGRPGERGERGE
KGERGEQGRDGPPGLPGTPGPPGPPGPKVSVDEPGPGLSGEQGPPGLKGAKGEPGSNGD
QGPKGDRGVPGIKGDRGEPGPRGQDGNPGLPGERGMAGPEGKPGLQGPRGPPGPVGGHG
DPGPPGAPGLAGPAGPQGPSGLKGEPGETGPPGRGLTGPTGAVGLPGPPGPSGLVGPQG
SPGLPGQVGETGKPGAPGRDGASGKDGDRGSPGVPGSPGLPGPVGPKGEPGPTGAPGQA
VVGLPGAKGEKGAPGGLAGDLVGEPGAKGDRGLPGPRGEKGEAGRAGEPGDPGEDGQKG
APGPKGFKGDPGVGVPGSPGPPGPPGVKGDLGLPGLPGAPGVVGFPGQTGPRGEMGQPG
PSGERGLAGPPGREGIPGPLGPPGPPGSVGPPGASGLKGDKGDPGVGLPGPRGERGEPG
IRGEDGRPGQEGPRGLTGPPGSRGERGEKGDVGSAGLKGDKGDSAVILGPPGPRGAKGD
MGERGPRGLDGDKGPRGDNGDPGDKGSKGEPGDKGSAGLPGLRGLLGPQGQPGAAGIPG
DPGSPGKDGVPGIRGEKGDVGFMGPRGLKGERGVKGACGLDGEKGDKGEAGPPGRPGLA
GHKGEMGEPGVPGQSGAPGKEGLIGPKGDRGFDGQPGPKGDQGEKGERGTPGIGGFPGP
SGNDGSAGPPGPPGSVGPRGPEGLQGQKGERGPPGERVVGAPGVPGAPGERGEQGRPGP
AGPRGEKGEAALTEDDIRGFVRQEMSQHCACQGQFIASGSRPLPSYAADTAGSQLHAVP
VLRVSHAEEEERVPPEDDEYSEYSEYSVEEYQDPEAPWDSDDPCSLPLDEGSCTAYTLR
WYHRAVTGSTEACHPFVYGGCGGNANRFGTREACERRCPPRVVQSQGTGTAQD*

In an embodiment, the COL7A1 gene comprises or consists of a nucleic acid sequence having 70%, 80%, 85%, 90%, 95%, 96%, 97%, 98% or 99% or more sequence identity with the nucleic acid sequence of SEQ ID NO: 1. In a different embodiment, the COL7A1 gene comprises or consists of a nucleic acid sequence wherein 0 to 30, 0 to 20, 0 to 10, 0 to 5, 0 to 3, 0 to 2 or 0 to 1 base(s) is deleted, substituted, or added with respect to the nucleic acid sequence of SEQ ID NO: 1. In a further embodiment, the COL7A1 gene comprises or consists of the nucleic acid sequence of SEQ ID NO: 1.

In an embodiment, the type VII collagen comprises or consists of an amino acid sequence having 70%, 80%, 85%, 90%, 95%, 96%, 97%, 98% or 99% or more sequence identity with the amino acid sequence of SEQ ID NO: 2. In a different embodiment, the type VII collagen comprises or consists of an amino acid sequence wherein 0 to 30, 0 to 20, 0 to 10, 0 to 5, 0 to 3, 0 to 2 or 0 to 1 amino acid residue (s) is deleted, substituted, or added with respect to the amino acid sequence of SEQ ID NO: 2. In a further embodiment, the type VII collagen comprises or consists of the amino acid sequence of SEQ ID NO: 2.

In an embodiment, the COL7A1 gene comprises or consists of a nucleic acid sequence that encodes an amino acid sequence having 70%, 80%, 85%, 90%, 95%, 96%, 97%, 98% or 99% or more sequence identity with the amino acid sequence of SEQ ID NO: 2. In a different embodiment, the COL7A1 gene comprises or consists of a nucleic acid sequence that encodes an amino acid sequence wherein 0 to 30, 0 to 20, 0 to 10, 0 to 5, 0 to 3, 0 to 2 or 0 to 1 amino acid residue (s) is deleted, substituted, or added with respect to the amino acid sequence of SEQ ID NO: 2.

As used herein, the term "sequence identity" with respect to a nucleic acid sequence or an amino acid sequence means the proportion of bases or amino acid residues that match between two sequences that are optimally aligned (aligned to be maximally matched) over the entire region of the sequence to be compared. The sequence to be compared may have an addition or a deletion (eg, a gap) in the optimal alignment of the two sequences. The sequence identity can be calculated using a program such as FASTA, BLAST, or CLUSTAL W provided in a public database (for example, DDBJ (http://www.ddbj.nig.ac.jp)). Alternatively, the sequence identity can be obtained using a commercially available sequence analysis software (for example, Vector NTI® software, GENETYX® ver. 12).

The cell may be genetically modified by any method. In an embodiment, the cell is genetically modified by genome editing such as the CRISPR system (eg, CRISPR/Cas9, CRISPR/Cpf1), TALEN, or ZFN. In a different embodiment, the cell is genetically modified by a viral vector such as a retroviral vector, lentiviral vector, adenoviral vector, or adeno-associated viral vector. In a further embodiment, the cell is genetically modified by CRISPR/Cas9. In a further embodiment, the cell is genetically modified by a retroviral vector or a lentiviral vector.

In genome editing, causing cleavage in the genome and introducing a donor vector comprising a sequence of interest into the cell can insert the sequence of interest into the cleavage site of the genome. The sequence to be inserted into the genome can be a COL7A1 gene or a sequence to be replaced with a portion containing a mutation in the COL7A1 gene (for example, a partial sequence of a COL7A1 gene). In addition to the sequence of interest, the donor vector may comprise a regulatory sequence such as a promoter or enhancer that controls the expression of the sequence of interest, or other elements such as a drug resistance gene for cell selection, and also may comprise, at both ends, sequences homogeneous to both ends of the insertion site of the genome. The donor vector can be introduced into a desired site as a result of non-homologous end binding or homologous recombination. As the donor vector, a plasmid, an adeno-associated viral vector, an integrase-deficient lentiviral vector, or any of other viral vectors can be used.

In the CRISPR system, an endonuclease such as Cas9 or Cas12 (eg, Cas12a (also called Cpf1), Cas12b, Cas12e) recognizes a specific base sequence, called PAM sequence, and the double strand of the target DNA is cleaved by the action of the endonuclease. When the endonuclease is Cas9, it cleaves about 3-4 bases upstream of the PAM sequence. Examples of endonucleases include Cas9 of S. pyogenes, S. aureus, N. meningitidis, S. thermophilus, or T. denticola, and Cpfl of L. bacterium ND2006 or Acidaminococcus sp. BV3L6. The PAM sequence varies depending on the endonuclease, and the PAM sequence of Cas9 in S. pyogenes is NGG, for example. A gRNA comprises a sequence of about 20 bases upstream of the PAM sequence (target sequence) or a sequence complementary thereto on the 5' end side, and plays a role of recruiting an endonuclease to the target sequence. The sequences other than the target sequence (or a sequence complementary thereto) of a gRNA can be appropriately determined by those skilled in the art depending on the endonuclease to be used. A gRNA may comprises a crRNA (CRISPR RNA), which comprises the target sequence or a sequence complementary thereto and is responsible for the sequence specificity of the gRNA, and a tracrRNA (Trans-activating crRNA), which contributes to the formation of a complex with Cas9 by forming a double strand. The crRNA and tracrRNA may exist as separate molecules. When the endonuclease is Cpf1, the crRNA alone functions as a gRNA. In the present specification, a gRNA comprising elements necessary for the function as a gRNA on a single strand may be particularly referred to as a sgRNA. The gRNA sequence can be determined by a tool available for target sequence selection and gRNA design, such as CRISPRdirect (https://crispr.dbcls.jp/).

A vector comprising a nucleic acid sequence encoding a gRNA and a nucleic acid sequence encoding an endonuclease may be introduced into and expressed in a cell, or a gRNA and an endonuclease protein that have been prepared extracellularly may be introduced into a cell. The endonuclease may include a nuclear localization signal. The nucleic acid sequence encoding a gRNA and the nucleic acid sequence encoding an endonuclease may be present on different vectors. Methods for introducing the vector, gRNA, and endonuclease into a cell include, but are not limited to, lipofection, electroporation, microinjection, calcium phosphate method, and DEAE-dextran method.

The present disclosure provides a gRNA and a vector comprising a nucleic acid sequence encoding a gRNA that can be used for the introduction of a COL7A1 gene into the genome. In an embodiment, the gRNA comprises any of the sequences of SEQ ID NOs: 3-5 or a sequence complementary thereto.

In the case of viral vectors, a COL7A1 gene can be introduced into the genome of a cell when a retroviral vector or a lentiviral vector having integrase activity is used. Alternatively, an integrase-deficient retroviral or lentiviral vector may be used. Integrase-deficient vectors lack integrase activity, for example, due to a mutation in the integrase gene. When an integrase-deficient vector, or an adenoviral vector or an adeno-associated viral vector is used, the sequence incorporated into the vector is not usually introduced into the genome of a cell. For example, when a COL7A1 gene is incorporated into an integrase-deficient lentiviral vector or an adenoviral vector, type VII collagen is expressed from the COL7A1 gene of the vector existing in the cell (in the nucleus).

A viral vector comprises a sequence encoding a COL7A1 gene and may contain a regulatory sequence such as a promoter or enhancer that controls the expression of the COL7A1 gene and other elements such as a drug resistance gene for cell selection. A viral vector may be prepared by any method known in the art. For example, a retroviral or lentiviral vector can be prepared by introducing a viral vector plasmid comprising LTR sequences at both ends (5' LTR and 3' LTR), a packaging signal, and a sequence of interest into a packaging cell with one or more plasmid vectors expressing structural proteins of the virus, such as Gag, Pol, and Env, or into a packaging cell that expresses such structural proteins. Examples of packaging cells include, but are not limited to, 293T cells, 293 cells, HeLa cells, COS1 cells, and COS7 cells. The viral vector may be pseudotyped and may express an envelope protein such as the vesicular stomatitis virus G protein (VSV-G). The sequence of interest can be introduced into a target cell by infecting the target cell with a viral vector thus prepared.

In an embodiment, the viral vector is a lentiviral vector. Examples of lentiviral vectors include, but are not limited to, HIV (human immunodeficiency virus) (for example, HIV-1 and HIV-2), SIV (simian immunodeficiency virus), FIV (feline immunodeficiency virus), MVV (Maedi-Visna virus), EV1 (Maedi-Visna-like virus), EIAV (equine infectious anemia virus), and CAEV (caprine arthritis encephalitis virus). In an embodiment, the lentiviral vector is HIV.

As an example, a lentiviral vector can be prepared as follows. First, a viral vector plasmid encoding the viral genome, one or more plasmid vectors expressing Gag, Pol, and Rev (and optionally Tat), and one or more plasmid vectors expressing envelope proteins such as VSV-G are introduced into a packaging cell. The viral vector plasmid comprises LTR sequences at both ends (5' LTR and 3' LTR), a packaging signal, and a COL7A1 gene and a promoter that controls its expression (eg, CMV promoter, EF1$\alpha$ promoter, or hCEF promoter). The 5' LTR functions as a promoter that induces transcription of the viral RNA genome, but may be replaced with a different promoter, such as CMV promoter, to enhance the expression of the RNA genome. Within the cell, the viral RNA genome is transcribed from the vector plasmid and packaged to form a viral core. The viral core is transported to the cell membrane of the packaging cell, encapsulated in the cell membrane, and released as a viral particle from the packaging cell. The released virus particle can be recovered from the culture supernatant of the packaging cell. For example, the virus particle can be recovered by any of conventional purification methods such as centrifugation, filter filtration, and column purification. A lentiviral vector can also be prepared by using a kit such as Lentiviral High Titer Packaging Mix, Lenti-X™ Packaging Single Shots (Takara Bio Inc.), and ViraSafe™ Lentivirus Complete Expression System (Cell Biolabs Inc.). An adeno-associated viral vector can be prepared by using a kit such as AAVpro® Helper Free System (Takara Bio Inc.).

A cell into which a sequence of interest has been introduced can be detected by Southern blotting or PCR. The sequence of interest need only be introduced into at least one of the alleles.

In an embodiment of the composition of the present disclosure, the mesenchymal stem cell is the most abundant cell in the composition. In a further embodiment, the mesenchymal stem cell accounts for 70%, 80%, 85%, 90%, 95%, 96%, 97%, 98% or 99% or more of cells comprised in the composition. In a further embodiment, the composition of the present disclosure is substantially free of cells other than the mesenchymal stem cell. The expression "substantially free of cells other than the mesenchymal stem cell" means that the composition only comprises a cell obtained by a method that is usually understood by those skilled in the art to be a method for obtaining a mesenchymal stem cell.

The number of cells comprised in a composition is an amount required to exert a desired effect (also referred to herein as an effective amount), and it is appropriately determined by those skilled in the art in consideration of factors such as the age, body weight, and medical condition of the patient, the type of cells and method for genetic modification. The number of cells is not limited to, but can be, for example, 1 cell to $1 \times 10^7$ cells, $1 \times 10$ cells to $1 \times 10^7$ cells, $1 \times 10^2$ cells to $1 \times 10^7$ cells, $1 \times 10^3$ cells to $1 \times 10^7$ cells, $1 \times 10^4$ cells to $1 \times 10^7$ cells, $1 \times 10^5$ cells to $1 \times 10^7$ cells, $1 \times 10^5$ cells to $5 \times 10^6$ cells, $5 \times 10^5$ cells to $1 \times 10^6$ cells, or $1 \times 10^5$ cells to $1 \times 10^6$ cells. The composition may comprise a pharmaceutically acceptable carrier and/or an additive in addition to the cell. Examples of pharmaceutically acceptable carriers include water, medium, saline, infusion containing glucose, D-sorbitol, D-mannitol or others, and phosphate buffered saline (PBS). Examples of additives include solubilizers, stabilizers, and preservatives. The dosage form of the composition is not particularly limited to, but can be a parenteral preparation such as an injection. Examples of injections include solution injections, suspension injections, emulsion injections, and injections to be prepared before use. The composition may be frozen and may contain a cryoprotectant such as DMSO, glycerol, polyvinylpyrrolidone, polyethylene glycol, dextran, or sucrose.

The composition of the present disclosure can be administered systemically or topically. In an embodiment, the composition is administered to an affected area of a patient with dystrophic epidermolysis bullosa. As used herein, the affected area means a blister or an area in the vicinity of a blister. In a further embodiment, the composition is administered intradermally at the site of a blister or administered into a blister. In a further embodiment, the composition is administered into a blister. In the present specification, administration into a blister means administration to the space under the epidermis of a blister. The intrablister administration can reduce patient distress as compared to intradermal or subcutaneous administration, and type VII collagen can be well expressed near the basement membrane. The number of cells administered per site is an amount required to exert a desired effect (effective amount), and it is appropriately determined by those skilled in the art in consideration of factors such as the age, body weight, and medical condition of the patient, the type of cells, and method for genetic modification. The number of cells is not limited to, but can be for example, 1 cell to $1 \times 10^7$ cells, $1 \times 10$ cells to $1 \times 10^7$ cells, $1 \times 10^2$ cells to $1 \times 10^7$ cells, $1 \times 10^3$ cells to $1 \times 10^7$ cells, $1 \times 10^4$ cells to $1 \times 10^7$ cells, $1 \times 10^5$ cells to $1 \times 10^7$ cells, $1 \times 10^5$ cells to $5 \times 10^6$ cells, $5 \times 10^5$ cells to $1 \times$

$10^6$ cells, or $1 \times 10^5$ cells to $1 \times 10^6$ cells. In an embodiment, the number of cells to be administered per blister is 1 cell to $1 \times 10^7$ cells, $1 \times 10$ cells to $1 \times 10^7$ cells, $1 \times 10^2$ cells to $1 \times 10^7$ cells, $1 \times 10^3$ cells to $1 \times 10^7$ cells, $1 \times 10^4$ cells to $1 \times 10^7$ cells, $1 \times 10^5$ cells to $1 \times 10^7$ cells, $1 \times 10^5$ cells to $5 \times 10^6$ cells, $5 \times 10^5$ cells to $1 \times 10^6$ cells, or $1 \times 10^5$ cells to $1 \times 10^6$ cells. The amount to be administered per blister may be adjusted according to the size of a blister when the above amount is considered for a standard blister having a diameter of 7 to 8 mm when circularly approximated.

Exemplary embodiments of the present invention are described below.

[1] A composition for use in the treatment of dystrophic epidermolysis bullosa, comprising a cell obtained from a patient with dystrophic epidermolysis bullosa, wherein the cell is a mesenchymal stem cell and genetically modified to produce type VII collagen.

[2] The composition according to item 1, wherein the cell is genetically modified by introducing a COL7A1 gene.

[3] The composition according to item 2, wherein the COL7A1 gene is introduced into the genome of the cell.

[4] The composition according to item 2 or 3, wherein the COL7A1 gene comprises a nucleic acid sequence having 70%, 80%, 85%, 90%, 95%, 96%, 97%, 98% or 99% or more sequence identity with the nucleic acid sequence of SEQ ID NO: 1, or a nucleic acid sequence that encodes an amino acid sequence having 70%, 80%, 85%, 90%, 95%, 96%, 97%, 98% or 99% or more sequence identity with the amino acid sequence of SEQ ID NO: 2.

[5] The composition according to any one of items 1 to 4, wherein the mesenchymal stem cell is a bone marrow-derived mesenchymal stem cell.

[6] The composition according to any one of items 1 to 5, wherein the mesenchymal stem cell is the most abundant cell in the composition.

[7] The composition according to any one of items 1 to 6, wherein the mesenchymal stem cell accounts for 70%, 80%, 85%, 90%, 95%, 96%, 97%, 98% or 99% or more of cells comprised in the composition.

[8] The composition according to any one of items 1 to 7, wherein the composition does not substantially comprise cells other than the mesenchymal stem cell.

[9] The composition according to any one of items 1 to 8, which is to be administered to an affected area.

[10] The composition according to any one of items 1 to 9, which is to be administered into a blister.

[11] The composition according to any one of items 1 to 10, wherein the cell is genetically modified by genome editing.

[12] The composition according to item 11, wherein the genome editing is carried out by CRISPR/Cas9.

[13] The composition according to any one of items 1 to 10, wherein the cell is genetically modified by a viral vector.

[14] The composition according to item 13, wherein the viral vector is a retroviral vector or a lentiviral vector.

[15] The composition according to item 13 or 14, wherein the viral vector is a lentiviral vector.

[16] A composition for use in the treatment of dystrophic epidermolysis bullosa, comprising a cell that produces type VII collagen, wherein the composition is to be administered into a blister.

[17] The composition according to item 16, wherein the cell is a cell genetically modified to produce type VII collagen.

[18] The composition according to item 17, wherein the cell is genetically modified by introducing a COL7A1 gene.

[19] The composition according to item 18, wherein the COL7A1 gene is introduced into the genome of the cell.

[20] The composition according to item 18 or 19, wherein the COL7A1 gene comprises a nucleic acid sequence having 70%, 80%, 85%, 90%, 95%, 96%, 97%, 98% or 99% or more sequence identity with the nucleic acid sequence of SEQ ID NO: 1, or a nucleic acid sequence that encodes an amino acid sequence having 70%, 80%, 85%, 90%, 95%, 96%, 97%, 98% or 99% or more sequence identity with the amino acid sequence of SEQ ID NO: 2.

[21] The composition according to any one of items 17 to 20, wherein the cell is a cell obtained from a patient with dystrophic epidermolysis bullosa.

[22] The composition according to any one of items 16 to 21, wherein the cell is a cell obtained from bone marrow.

[23] The composition according to any one of items 16 to 22, wherein the cell is a mesenchymal stem cell.

[24] The composition according to item 23, wherein the mesenchymal stem cell is a bone marrow-derived mesenchymal stem cell.

[25] The composition according to item 23 or 24, wherein the mesenchymal stem cell is the most abundant cell in the composition.

[26] The composition according to any one of items 23 to 25, wherein the mesenchymal stem cell accounts for 70%, 80%, 85%, 90%, 95%, 96%, 97%, 98% or 99% or more of cells comprised in the composition.

[27] The composition according to any one of items 23 to 26, wherein the composition does not substantially comprise cells other than the mesenchymal stem cell.

[28] The composition according to any one of items 17 to 27, wherein the cell is genetically modified by genome editing.

[29] The composition according to item 28, wherein the genome editing is carried out by CRISPR/Cas9.

[30] The composition according to any one of items 17 to 27, wherein the cell is genetically modified by a viral vector.

[31] The composition according to item 30, wherein the viral vector is a retroviral vector or a lentiviral vector.

[32] The composition according to item 30 or 31, wherein the viral vector is a lentiviral vector.

[33] A method of producing a composition for use in the treatment of dystrophic epidermolysis bullosa, comprising genetically modifying a cell to produce type VII collagen, and,
preparing a composition comprising the genetically modified cell.
[34] A method of treating dystrophic epidermolysis bullosa, comprising administering to a patient of dystrophic epidermolysis bullosa a composition comprising a cell that produces type VII collagen.
[35] The method according to item 34, wherein the cell is genetically modified to produce type VII collagen.
[36] The method according to item 35, wherein the cell is genetically modified by introducing a COL7A1 gene.
[37] The method according to item 36, wherein the COL7A1 gene is introduced into the genome of the cell.
[38] The composition according to item 36 or 37, wherein the COL7A1 gene comprises a nucleic acid sequence having 70%, 80%, 85%, 90%, 95%, 96%, 97%, 98% or 99% or more sequence identity with the nucleic acid sequence of SEQ ID NO: 1, or a nucleic acid sequence that encodes an amino acid sequence having 70%, 80%, 85%, 90%, 95%, 96%, 97%, 98% or 99% or more sequence identity with the amino acid sequence of SEQ ID NO: 2.
[39] The method according to any one of items 35 to 38, wherein the cell is genetically modified by genome editing.
[40] The method according to item 39, wherein the genome editing is carried out by CRISPR/Cas9.
[41] The method according to any one of items 35 to 38, wherein the cell is genetically modified by a viral vector.
[42] The method according to item 41, wherein the viral vector is a retroviral vector or a lentiviral vector.
[43] The method according to item 41 or 42, wherein the viral vector is a lentiviral vector.
[44] The method according to any one of items 35 to 43, further comprising, prior to the adminstering to the patient, genetically modifying a cell to produce type VII collagen.
[45] The method according to item 33 or 44, comprising genetically modifying the cell by introducting a COL7A1 gene.
[46] The method according to item 45, comprising introducting the COL7A1 gene into the genome of the cell.
[47] The method according to item 45 or 46, wherein the COL7A1 gene comprises a nucleic acid sequence having 70%, 80%, 85%, 90%, 95%, 96%, 97%, 98% or 99% or more sequence identity with the nucleic acid sequence of SEQ ID NO: 1, or a nucleic acid sequence that encodes an amino acid sequence having 70%, 80%, 85%, 90%, 95%, 96%, 97%, 98% or 99% or more sequence identity with the amino acid sequence of SEQ ID NO: 2.
[48] The method according to any one of items 33 and 44 to 47, comprising genetically modifying the cell by genome editing.
[49] The method according to item 48, wherein the genome editing is carried out by CRISPR/Cas9.
[50] The method according to any one of items 33 and 44 to 47, comprising genetically modifying the cell with a viral vector.
[51] The method according to item 50, wherein the viral vector is a retroviral vector or a lentiviral vector.
[52] The method according to item 50 or 51, wherein the viral vector is a lentiviral vector.
[53] The method according to any one of items 33 to 52, wherein the cell is a cell obtained from a patient with dystrophic epidermolysis bullosa.
[54] The method according to any one of items 33 and 44 to 53, further comprising, prior to the genetically modifying, obtaining a cell from a patient with dystrophic epidermolysis bullosa.
[55] The method according to any one of items 33 to 54, wherein the cell is a cell obtained from bone marrow.
[56] The method according to any one of items 33 to 55, wherein the cell is a mesenchymal stem cell.
[57] The method according to item 56, wherein the mesenchymal stem cell is a bone marrow-derived mesenchymal stem cell.
[58] The method according to item 56 or 57, wherein the mesenchymal stem cell is the most abundant cell in the composition.
[59] The method according to any one of items 56 to 58, wherein the mesenchymal stem cell accounts for 70%, 80%, 85%, 90%, 95%, 96%, 97%, 98% or 99% or more of cells comprised in the composition.
[60] The method according to any one of items 56 to 59, wherein the composition does not substantially comprise cells other than the mesenchymal stem cell.
[61] The method according to any one of itmes 34 to 60, comprising administreing the composition to an affected area.
[62] The method according to any one of itmes 34 to 61, comprising administreing the composition into a blister.
[63] Use of a composition comprising a cell obtained from a patient with dystrophic epidermolysis bullosa for the manufacture of a medicament for treating dystrophic epidermolysis bullosa, wherein the cell comprises a mesenchymal stem cell and the cell is genetically modified to produce type VII collagen.
[64] Use of a composition comprising a cell that produces type VII collagen for the manufacture of a medicament for treating dystrophic epidermolysis bullosa, wherein the composition is to be administered into a blister.
[65] Use of a composition copmrising a cell obtained from a patient with dystrophic epidermolysis bullosa for treating dystrophic epidermolysis bullosa, wherein the cell comprises a mesenchymal stem cell and the cell is genetically modified to produce type VII collagen.
[66] Use of a composition copmrising a cell that produces type VII collagen for treating dystrophic epidermolysis bullosa, wherein the composition is to be administered into a blister.

[67] A gRNA comprising a sequence of any one of SEQ ID NOs: 3 to 5 or a sequence complementary thereto.
[68] A vector copmrising a nucleic acid sequence encoding the gRNA of item 67.

The present invention is described in more detail with reference to the examples hereinafter, but not limited to the embodiments described below.

Examples

1. Design of genome editing

Three types of sgRNAs were prepared in order to select a site with good cleavage efficiency by the CRISPR-Cas9 system in the AAVS1 (Adeno-associated virus integration site 1) region in the human genome. The AAVS1 region is a safe region that is not easily affected by gene transfer (safe harbor). Since the CRISPR-Cas9 system recognized the base sequence of "NGG" and cleaved 3 bases upstream of the sequence, regions each having "GG" at the end were selected and sgRNAs each containing a sequence of 20 bases upstream of "NGG" were designed (sgAAVS1-#1 to #3) (Fig. 1, top; Table 1).

Table 1

| sgRNA | Target sequence | SEQ ID NO. |
|---|---|---|
| sgAAVS1-#1 | ACCCCACAGTGGGGCCACTA | 3 |
| sgAAVS1-#2 | GTCACCAATCCTGTCCCTAG | 4 |
| sgAAVS1-#3 | GGGGCCACTAGGGACAGGAT | 5 |

An oligonucleotide consisting of a sequence of any one of SEQ ID NOs: 3 to 5 was annealed with its complementary strand and cloned into the Bbs1 site of eSpCas9 (1.1) (Addgene plasmid # 71814). This plasmid (2.5 pg) was introduced into HEK293 cells (human fetal kidney cell line) seeded in 6-well dishes by Lipofectamin 3000 (Thermo Fisher Scientific). Forty-eight hours after transfection, genomic DNA was extracted from the cells and the region containing the target site was amplified by PCR. The PCR amplified fragments were subjected to heat treatment to be a single chain, annealed by slow cooling, and then treated with a mismatch site-specific endonuclease. The resulting product was fractionated by electrophoresis, the degree of insertion or deletion mutation introduced by the genome cleavage was measured from the density of the band, and the genome editing efficiency was calculated by the following formula (In the formula, "a" indicates the concentration of the band that was not digested, and "b" and "c" indicate the concentrations of the cleaved bands.).

$$\mathrm{Indel}\ (\%) = 100 \times \left(1 - \sqrt{(1 - f\mathrm{cut})}\right), \quad f_{\mathrm{cut}} = (\mathrm{b}+\mathrm{c}) / (\mathrm{a}+\mathrm{b}+\mathrm{c})$$

All sgRNAs of sgAAVS1-#1 to #3 produced a short DNA fragment different from the control, confirming that double-strand break occured (Fig. 1, bottom). In the following experiments, sgAAVS1-#3, which had the highest cleavage efficiency, was used.

2. Introduction of COL7A1 gene into BM-MSCs

For the introduction of a COL7A1 gene into the AAVS1 region, a plasmid expressing the COL7A1 gene under the control of the CAG promoter was designed (Fig. 2). The COL7A1 cDNA was obtained from Flexi ORF sequence-verified clone (Promega, Madison, WI, USA). The COL7A1 cDNA was subcloned into the pENTR1A plasmid (ThermoFisher Scientific) to prepare pENTR1A-COL7A1, and pAAVS-P-CAG-COL7A1 was obtained by using LR recombinase (ThermoFisher Scientific) and pAAVS-P-CAG-DEST (Addgene plasmid # 80490) and pENTR1A-COL7A1.

Since the transfer efficiency increased but the cell viability decreased as the amount of plasmid increased with respect to the cells, first, the experimental conditions in which both the cell viability and the transfer efficiency were good were examined. To human bone marrow-derived mesenchymal stem cells (BM-MSCs) [PromoCell (Heidelberg, Germany) or Lonza (Basel, Switzerland)] ($1 \times 10^5$ cells), pAAVS-P-CAG-COL7A1 (0 μg, 0.25 μg, 0.5 μg or 1.0 μg) and eSpCas9 (1.1) expressing sgAAVS1-#3 (0 μg, 0.25 μg, 0.5 μg, or 1.0 μg) were introduced by electroporation. All cells were collected 24 hours after transfection, and the viability was calculated from the number of trypan blue staining-positive cells (dead cells) with respect to the total number of cells. In addition, 48 hours after transfection, BM-MSCs were cultured in a medium containing 0.5 μg/mL puromycin (Invivogen, San Diego, CA, USA) for about 2 weeks for selection, and the number of isolated colonies was determined to measure the efficiency of genome editing. Based on the results shown in Fig. 3, 0.25 μg of pAAVS-P-CAG-COL7A1 and 0.5 μg of eSpCas9 (1.1) were used in the following experiments.

To BM-MSCs ($1 \times 10^5$ cells), pAAVS-P-CAG-COL7A1 (0.25 pg) and eSpCas9 (1.1) expressing sgAAVS1-#3 (0.5 μg) were introduced by electroporation. Forty-eight hours after transfection, BM-MSCs were cultured in a medium containing 0.5 μg/mL puromycin (Invivogen, San Diego, CA, USA) for about 2 weeks for selection. Genomic DNA was extracted from the BM-MSCs, and genome editing and introduction of the COL7A1 gene were confirmed by PCR.

The PCR product was obtained by amplification between F1-R1, confirming that genome editing had occurred (Fig. 4, F1-R1). Also, long DNA not present in the wild type (WT) was detected in the PCR product between F2-R2, and it indicated that the COL7A1 gene was introduced (Fig. 4, F2-R2). The COL7A1 gene was introduced into one of the alleles (Fig. 4, Monoallelic) or both (Fig. 4, Biallelic).

Since collagen was a secretory protein and exuded to the outside of cells, expression of type VII collagen in BM-MSCs was observed by immunostaining with an anti-type VII collagen antibody (Sigma Aldrich, St. Louis, MO, USA) and western blotting of the culture supernatant. As shown in Fig. 5, the expression of type VII collagen was confirmed in the genetically modified MSCs.

3. Expression of type VII collagen in epidermolysis bullosa model mice

Expression of type VII collagen in epidermolysis bullosa model mice that received genetically modified MSCs was examined. The full-thickness skin of a neonatal Col7A1 gene knockout mouse (Col7a1-/-) showing blistering was excised and transplanted to the back of an immunodeficient mouse (NOD-SCID). One week after transplantation, the genetically modified MSCs described in section 2 above were injected subcutaneously or intradermally with 0.1 to $1.0 \times 10^6$ cells (Fig. 6). For intrablister injection, immediately after transplantation, the skin surface was pinched and rubbed to form blisters, and 0.1 to $1.0 \times 10^6$ cells of genetically modified MSCs were immediately injected into the space under the epidermis. Four weeks after each injection, the transplanted skin was excised, and the expression of type VII collagen was evaluated by immunostaining with an anti-type VII collagen antibody (Sigma Aldrich, St. Louis, MO, USA).

As shown in Fig. 7, the expression of type VII collagen was observed near the basement membrane in the intradermal injection and the intrablister injection, but the expression in the intradermal injection was partial. In the subcutaneous injection, collagen was expressed in a deep layer different from the basement membrane (Fig. 8). These results show that an excellent therapeutic effect is expected especially by intrablister injection.

Further, in the same manner as above, $2.0 \times 10^6$ cells of the genetically modified MSCs described in section 2 above was injected into blisters. Four weeks after that, the transplanted skin was excised and observed with an electron microscope. Formation of anchoring fibrils was confirmed (Fig. 9).

## Claims

1. A composition for use in the treatment of dystrophic epidermolysis bullosa, comprising a cell obtained from a patient with dystrophic epidermolysis bullosa, wherein the cell is a mesenchymal stem cell and genetically modified to produce type VII collagen.

2. The composition according to claim 1, wherein the cell is genetically modified by introducing a COL7A1 gene.

3. The composition according to claim 2, wherein the COL7A1 gene comprises a nucleic acid sequence having 90% or more sequence identity with the nucleic acid sequence of SEQ ID NO: 1, or a nucleic acid sequence that encodes an amino acid sequence having 90% or more sequence identity with the amino acid sequence of SEQ ID NO: 2.

4. The composition according to any one of claims 1 to 3, wherein the mesenchymal stem cell is a bone marrow-derived mesenchymal stem cell.

5. The composition according to any one of claims 1 to 4, wherein the mesenchymal stem cell is the most abundant cell in the composition.

6. The composition according to any one of claims 1 to 5, which is to be administered into a blister.

7. A composition for use in the treatment of dystrophic epidermolysis bullosa, comprising a cell that produces type VII collagen, wherein the composition is to be administered into a blister.

8. The composition according to claim 7, wherein the cell is a cell genetically modified to produce type VII collagen.

9. The composition according to claim 8, wherein the cell is genetically modified by introducing a COL7A1 gene.

10. The composition according to claim 9, wherein the COL7A1 gene comprises a nucleic acid sequence having 90% or more sequence identity with the nucleic acid sequence of SEQ ID NO: 1, or a nucleic acid sequence that encodes an amino acid sequence having 90% or more sequence identity with the amino acid sequence of SEQ ID NO: 2.

11. The composition according to any one of claims 8 to 10, wherein the cell is a cell obtained from a patient with dystrophic epidermolysis bullosa.

12. The composition according to any one of claims 7 to 11, wherein the cell is a cell obtained from bone marrow.

13. The composition according to any one of claims 7 to 12, wherein the cell is a mesenchymal stem cell.

14. The composition according to claim 13, wherein the mesenchymal stem cell is a bone marrow-derived mesenchymal stem cell.

15. The composition according to claim 13 or 14, wherein the mesenchymal stem cell is the most abundant cell in the composition.

16. A gRNA comprising a sequence of any one of SEQ ID NOs: 3 to 5 or a sequence complementary thereto.

Fig. 1

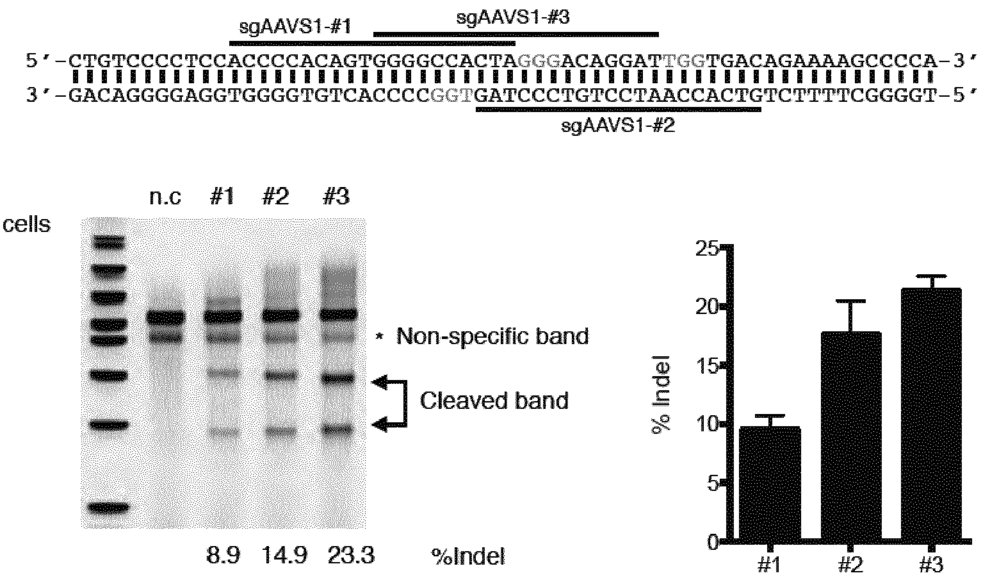
sgAAVS1-#1
sgAAVS1-#3
5'-CTGTCCCCTCCACCCCACAGTGGGGCCACTAGGGACAGGATTGGTGACAGAAAAGCCCCA-3'
3'-GACAGGGGAGGTGGGGTGTCACCCCGGTGATCCCTGTCCTAACCACTGTCTTTTCGGGGT-5'
sgAAVS1-#2
293T cells
n.c
#1
#2
#3
* Non-specific band
Cleaved band
8.9
14.9
23.3
%Indel
% Indel
25
20
15
10
5
0
#1
#2
#3

Fig. 2

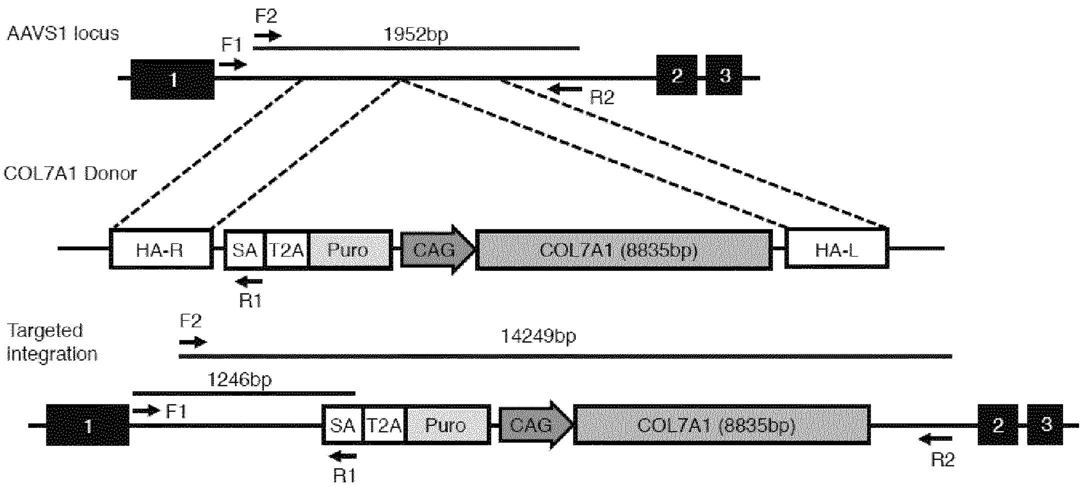
AAVS1 locus
F2
F1
1952bp
1
2
3
R2
COL7A1 Donor
HA-R
SA
T2A
Puro
CAG
COL7A1 (8835bp)
HA-L
R1
Targeted integration
F2
14249bp
1246bp
1
F1
SA
T2A
Puro
CAG
COL7A1 (8835bp)
2
3
R1
R2

Fig. 3

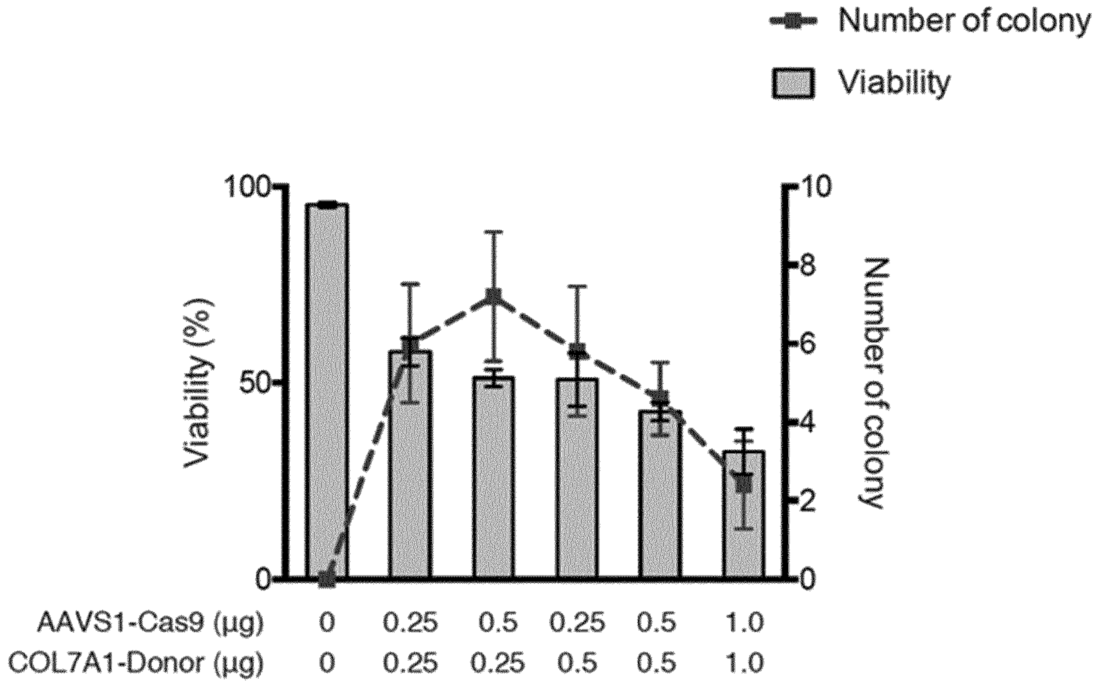
Number of colony
Viability
100
50
0
Viability (%)
10
8
6
4
2
0
Number of colony
AAVS1-Cas9 (µg) 0 0.25 0.5 0.25 0.5 1.0
COL7A1-Donor (µg) 0 0.25 0.25 0.5 0.5 1.0

Fig. 4

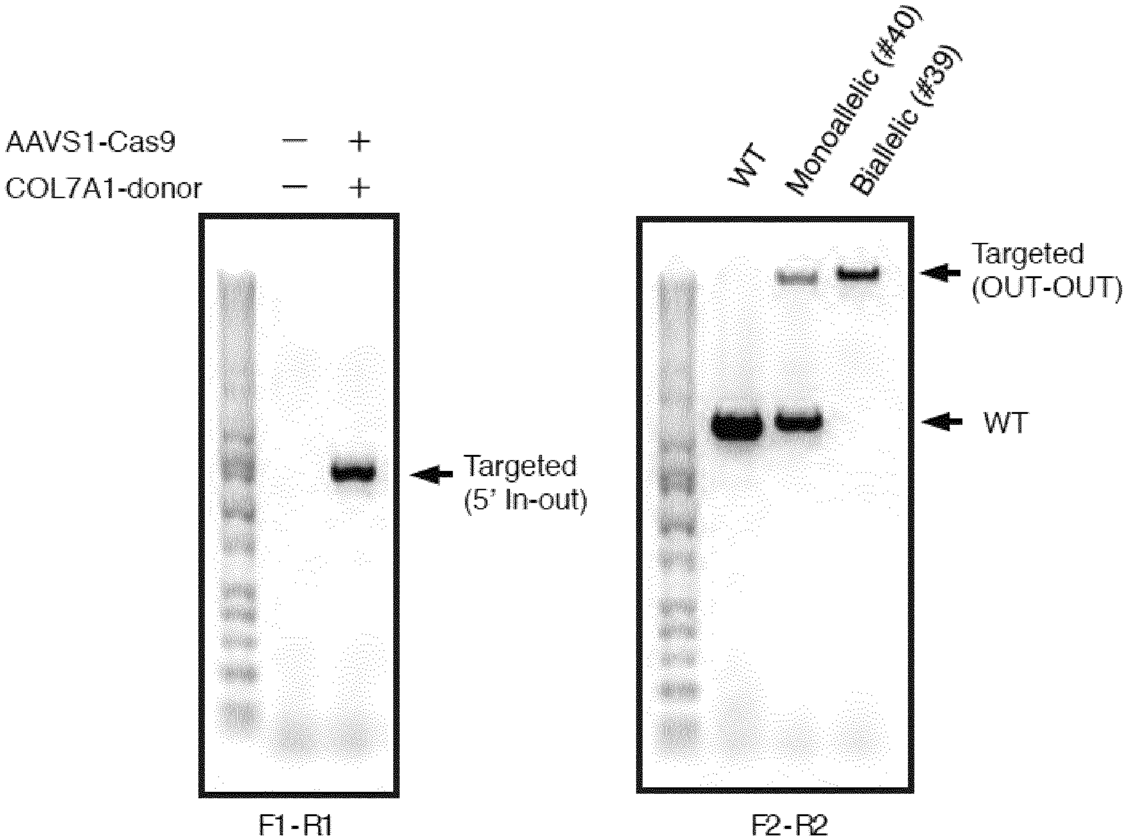
AAVS1-Cas9 — +
COL7A1-donor — +
Targeted (5' In-out)
F1-R1
WT
Monoallelic (#40)
Biallelic (#39)
Targeted (OUT-OUT)
WT
F2-R2

Fig. 5

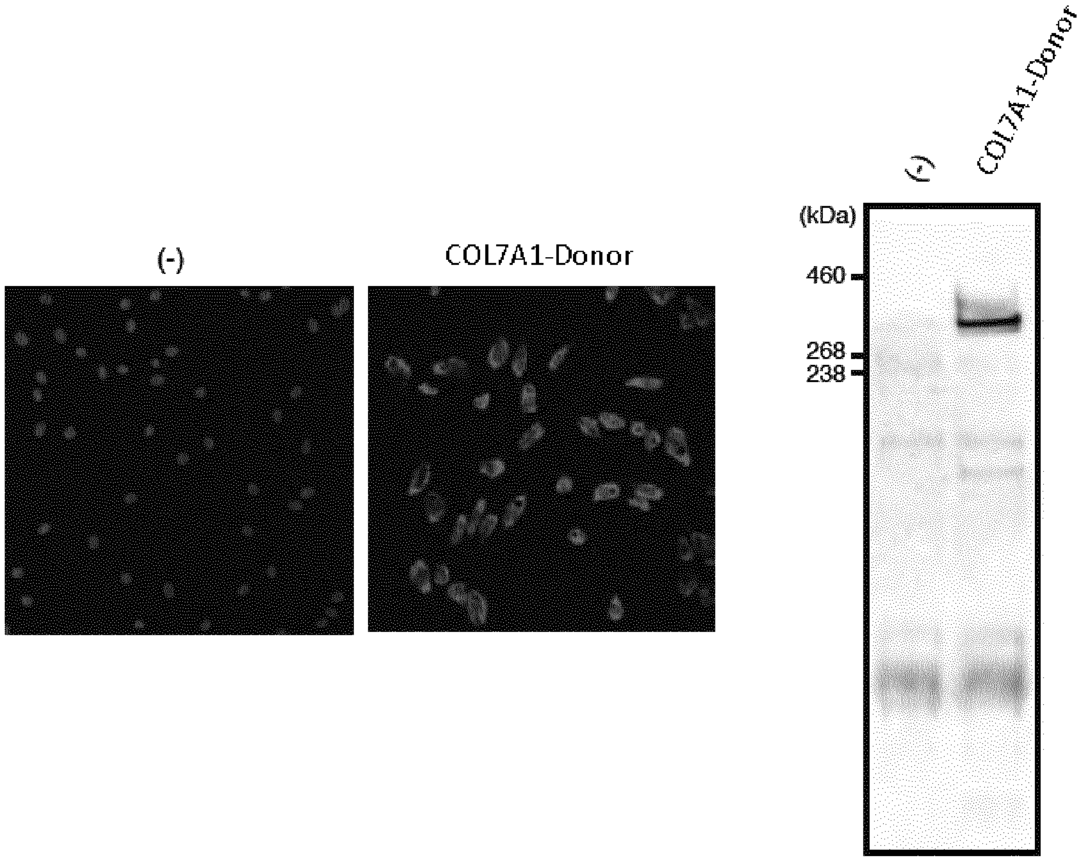
(-)
COL7A1-Donor
(kDa)
460
268
238

Fig. 6

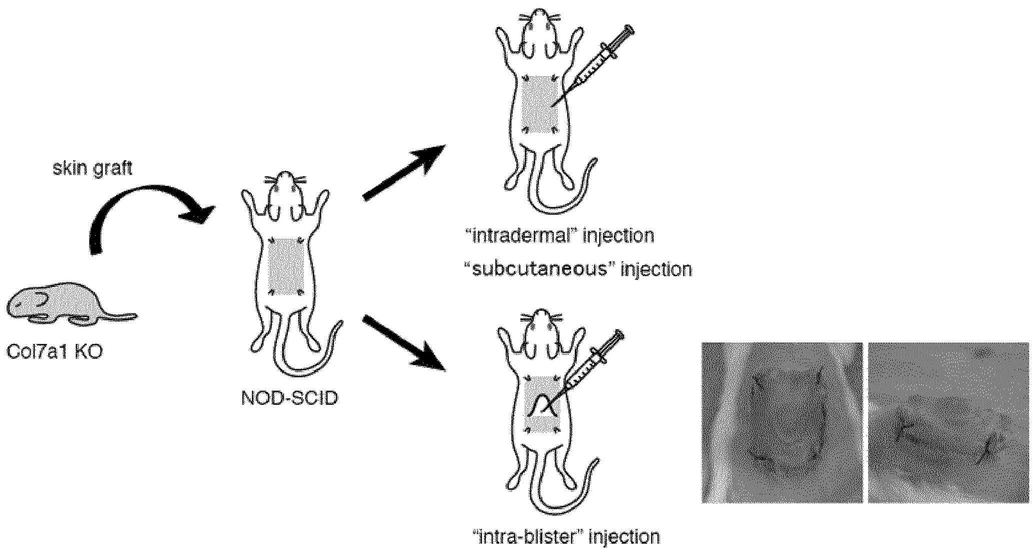
skin graft
Col7a1 KO
NOD-SCID
"intradermal" injection
"subcutaneous" injection
"intra-blister" injection

Fig. 7

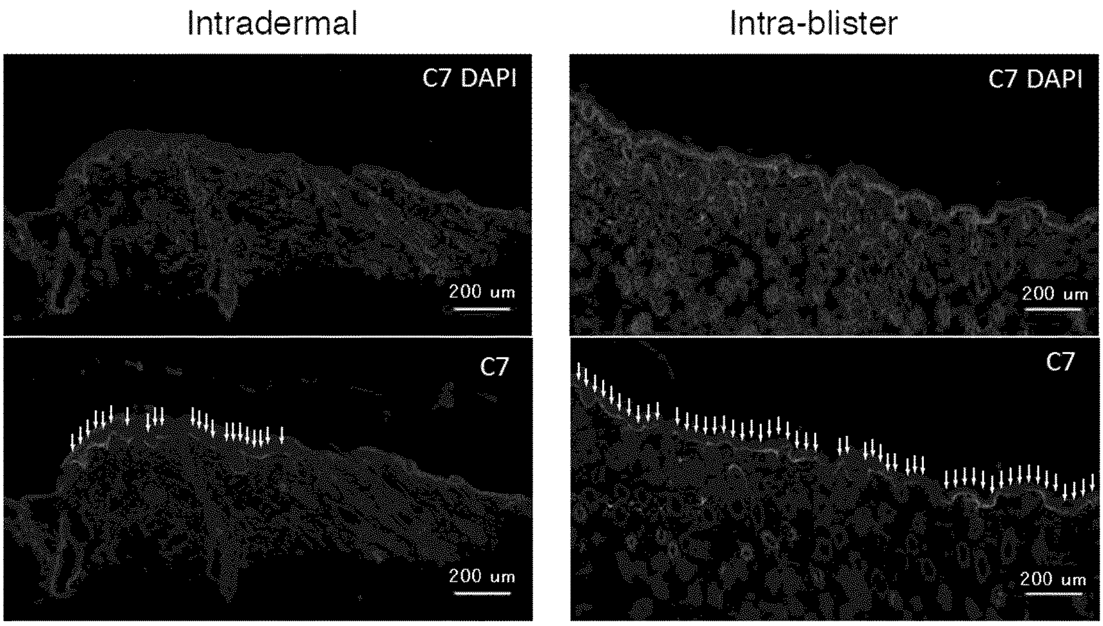
Intradermal
Intra-blister
C7 DAPI
200 um
C7 DAPI
200 um
C7
200 um
C7
200 um

Fig. 8

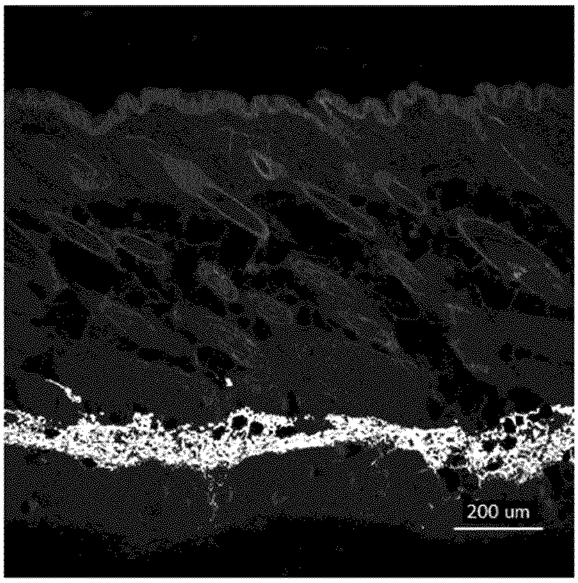
200 um

Fig. 9

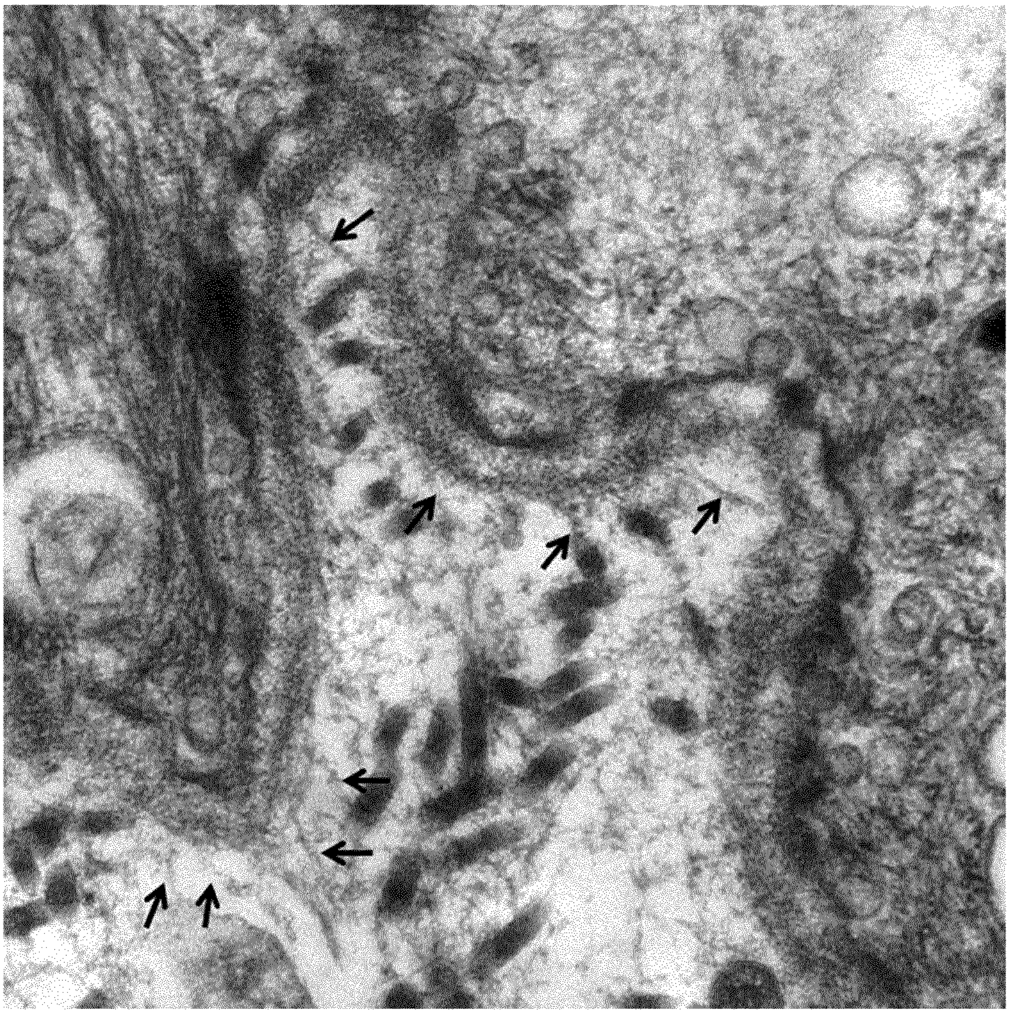

**INTERNATIONAL SEARCH REPORT**

International application No.
PCT/JP2020/001433

A. CLASSIFICATION OF SUBJECT MATTER
A61K 48/00(2006.01)i; A61P 17/00(2006.01)i; A61P 17/02(2006.01)i; C12N 5/0775(2010.01)i; C12N 5/22(2006.01)i; C12N 15/12(2006.01)i; A61K 38/39(2006.01)i; A61K 35/28(2015.01)i; A61K 35/32(2015.01)i
FI: A61K35/28; A61K48/00; A61P17/00; A61P17/02; A61K38/39; A61K35/32; C12N5/22; C12N5/0775; C12N15/12

According to International Patent Classification (IPC) or to both national classification and IPC

B. FIELDS SEARCHED

Minimum documentation searched (classification system followed by classification symbols)
A61K48/00; A61P17/00; A61P17/02; A61K35/28; A61K35/32; C12N5/0775; C12N5/22; C12N15/12; A61K38/39

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII);
CAplus/RECISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2016-521975 A (SANGAMO BIOSCIENCES, INC.) 28.07.2016 (2016-07-28) table 1, SEQ ID NO: 165 | 16<br>16<br>1-15 |
| X<br>Y<br>A | CN 107904208 A (YUNZHOU BIOSCIENCES (GUANGZHOU) INC.) 13.04.2018 (2018-04-13) paragraph [0062], SEQ. ID No. 6 | 16<br>16<br>1-15 |
| Y<br>A | JP 2015-516162 A (SANGAMO BIOSCIENCES, INC.) 11.06.2015 (2015-06-11) paragraphs [0060], [0090], [0095], [0153] | 16<br>1-15 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 March 2020 (09.03.2020) | 31 March 2020 (31.03.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2020/001433

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 2011-518555 A (SANGAMO BIOSCIENCES, INC.) 30.06.2011 (2011-06-30) example 1, table 1 | 16<br>1-15 |
| X<br>Y<br>A | WO 2017/161180 A1 (INTREXON CORPORATION) 21.09.2017 (2017-09-21) claims, examples 6, 8 | 7-9,11<br>1-15<br>16 |
| Y<br>A | US 2018/0142262 A1 (REGENTS OF THE UNIVERSITY OF MINNESOTA) 24.05.2018 (2018-05-24) paragraphs [0004]-[0008], [0047]-[0052], [0065], [0071], [0075], [0076], [0086], [0125], [0126] | 1-15<br>16 |
| Y<br>A | KUHL, T. et al., "High Local Concentrations of Intradermal MSCs Restore Ski Integrity and Facilitate Wound Healing in Dystrophic Epidermolysis Bullosa", Mol. Ther., 2015, vol. 23, no. 8, pp. 1368-1379, doi: 10.1038/mt.2015.58, abstract, page 1368, right column to page 1369, page 1372, left column, paragraph [0003] to page 1373, left column, paragraph [0001], page 1377, right column, paragraph [0002] | 1-15<br>16 |
| Y<br>A | 玉井 克人, 表皮水疱症に対する再生医療と遺伝子治療：現状と展望, 医学のあゆみ, 05 May 2018, vol. 265, no. 5, pp. 463-468, page 465, right column, last paragraph, (TAMAI, Katsuto, "Regenerative therapy and gene therapy for epidermolysis bullosa : current status and future perspective", Journal of clinical and experimental medicine) | 1-15<br>16 |
| Y<br>A | EL-DAROUTI, M. et al., "Treatment of dystrophic epidermolysis bullosa with bone marrow non-hematopoietic stem cells: a randomized controlled trial", Dermatologic Therapy, 2016, vol. 29, pp. 96-100, abstract, page 97, left column, page 99, left column | 1-15<br>16 |
| P,X<br>P,Y<br>P,A | PETROVA, A. et al., "Human Mesenchymal Stromal cells Engineered to Express Collagen VII Can Restore Anchoring Fibrils in Recessive Dystrophic Epidermolysis Bullosa Skin Graft Chimeras", Journal of Investigative Dermatology, January 2020, vol. 140, no. 1, pp. 121-131, published online 03 October 2019, abstract, page 122, left column, paragraph [0003] to right column, pp. 124-126 | 1,2,4-9,11,13,15<br>1-15<br>16 |
| P,X<br>P,A | WO 2019/210042 A1 (SEATTLE CHILDREN'S HOSPITAL) 31.10.2019 (2019-10-31) SEQ ID NO. 17 | 16<br>1-15 |

Form PCT/ISA/210 (continuation of second sheet) (January 2015)

INTERNATIONAL SEARCH REPORT

International application No.
PCT/JP2020/001433

C (Continuation). DOCUMENTS CONSIDERED TO BE RELEVANT

| Category* | Citation of document, with indication, where appropriate, of the relevant passages | Relevant to claim No. |
|---|---|---|
| A | CONGET, P. et al., "Replenishment of type VII collagen and re-epithelialization of chronically ulcerated skin after intradermal administration of allogeneic mesenchymal stromal cells in two patients with recessive dystrophic epidermolysis bullosa", Cytotherapy, 2010, vol. 12, pp. 429-431, abstract | 1-16 |
| A | WO 2018/186480 A (STEMRIM INC.) 11.10.2018 (2018-10-11) claims, abstract | 1-16 |
| A | WO 2018/235834 A1 (NATIONAL UNIVERSITY CORPORATION HOKKAIDO UNIVERSITY) 27.12.2018 (2018-12-27) claims, abstract | 1-16 |
| A | WO 2018/154413 A1 (CRISPR THERAPEUTICS AG) 30.08.2018 (2018-08-30) claims, paragraphs [0006]-[0009], [0025], [0026] | 1-16 |

Form PCT/ISA/210 (continuation of second sheet) (January 2015)

**INTERNATIONAL SEARCH REPORT**
Information on patent family members

International application No.
PCT/JP2020/001433

| Patent Documents referred in the Report | Publication Date | Patent Family | Publication Date |
|---|---|---|---|
| JP 2016-521975 A | 28 Jul. 2016 | WO 2014/186585 A2<br>table 1, SEQ No. 165<br>US 2015/0056705 A1<br>EP 2997146 A2<br>CN 105683376 A | |
| CN 107904208 A | 13 Apr. 2018 | WO 2019/127116 A1<br>page 8, SEQ. ID No. 6 | |
| JP 2015-516162 A | 11 Jun. 2015 | WO 2013/169802 A1<br>paragraphs [0101], [0131], [0136], [0194]<br>US 2013/0326645 A1<br>EP 2847338 A1<br>KR 10-2015-0006469 A<br>CN 104471067 A | |
| JP 2011-518555 A | 30 Jun. 2011 | WO 2009/131632 A1<br>example 1, table 1<br>US 2009/0263900 A1<br>EP 2281050 A1 | |
| WO 2017/161180 A1 | 21 Sep. 2017 | JP 2019-508454 A<br>claims, examples 6, 8<br>US 2019/0192636 A1<br>EP 3429609 A1<br>KR 10-2018-0118795 A<br>CN 109257926 A | |
| US 2018/0142262 A1 | 24 May 2018 | (Family: none) | |
| WO 2019/210042 A1 | 31 Oct. 2019 | (Family: none) | |
| WO 2018/186480 A1 | 11 Oct. 2018 | CA 3058877 A1<br>claims, abstract | |
| WO 2018/235834 A1 | 27 Dec. 2018 | (Family: none) | |
| WO 2018/154413 A1 | 30 Aug. 2018 | US 2019/0365929 A1<br>claims, paragraphs [0006]-[0009], [0025], [0026] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019007201 A
- WO 2017120147 A
- WO 2018154413 A


### Non-patent literature cited in the description


- **BUDNIATZKY ; GEPSTEIN.** *Stem Cells Transl Med,* 2014, vol. 3 (4), 448-57
- **BARRETT et al.** *Stem Cells Trans Med,* 2014, vol. 3, 1-6
- **FOCOSI et al.** *Blood Cancer Journal,* 2014, vol. 4, e211

SEQUENCE LISTING

<110> OSAKA UNIVERSITY

<120> Composition for use in treating dystrophic epidermolysis bullosa

<130> 675347

<150> JP 2019-007201
<151> 2019-01-18

<160> 5

<170> PatentIn version 3.5

<210> 1
<211> 8835
<212> DNA
<213> Homo sapiens

<400> 1
atgacgctgc ggcttctggt ggccgcgctc tgcgccggga tcctggcaga ggcgccccga 60

gtgcgagccc agcacaggga gagagtgacc tgcacgcgcc tttacgccgc tgacattgtg 120

ttcttactgg atggctcctc atccattggc cgcagcaatt tccgcgaggt ccgcagcttt 180

ctcgaagggc tggtgctgcc tttctctgga gcagccagtg cacagggtgt gcgctttgcc 240

acagtgcagt acagcgatga tccacggaca gagttcggcc tggatgcact tggctctggg 300

ggtgatgtga tccgcgccat ccgtgagctt agctacaagg ggggcaacac tcgcacaggg 360

gctgcaattc tccatgtggc tgaccatgtc ttcctgcccc agctggcccg acctggtgtc 420

cccaaggtct gcatcctgat cacagacggg aagtcccagg acctggtgga cacagctgcc 480

caaaggctga aggggcaggg ggtcaagcta tttgctgtgg ggatcaagaa tgctgaccct 540

gaggagctga agcgagttgc ctcacagccc accagtgact tcttcttctt cgtcaatgac 600

ttcagcatct tgaggacact actgcccctc gtttcccgga gagtgtgcac gactgctggt 660

ggcgtgcctg tgacccgacc tccggatgac tcgacctctg ctccacgaga cctggtgctg 720

tctgagccaa gcagccaatc cttgagagta cagtggacag cggccagtgg ccctgtgact 780

ggctacaagg tccagtacac tcctctgacg gggctgggac agccactgcc gagtgagcgg 840

caggaggtga acgtcccagc tggtgagacc agtgtgcggc tgcggggtct ccggccactg 900

accgagtacc aagtgactgt gattgccctc tacgccaaca gcatcgggga ggctgtgagc 960

gggacagctc ggaccactgc cctagaaggg ccggaactga ccatccagaa taccacagcc 1020

cacagcctcc tggtggcctg gcggagtgtg ccaggtgcca ctggctaccg tgtgacatgg 1080

cgggtcctca gtggtgggcc cacacagcag caggagctgg gccctgggca gggttcagtg 1140

ttgctgcgtg acttggagcc tggcacggac tatgaggtga ccgtgagcac cctatttggc 1200

cgcagtgtgg ggcccgccac ttccctgatg gctcgcactg acgcttctgt tgagcagacc 1260

ctgcgcccgg tcatcctggg ccccacatcc atcctccttt cctggaactt ggtgcctgag 1320

gcccgtggct accggttgga atggcggcgt gagactggct tggagccacc gcagaaggtg 1380

gtactgccct ctgatgtgac ccgctaccag ttggatgggc tgcagccggg cactgagtac 1440

cgcctcacac tctacactct gctggagggc cacgaggtgg ccacccctgc aaccgtggtt 1500

cccactggac cagagctgcc tgtgagccct gtaacagacc tgcaagccac cgagctgccc 1560

gggcagcggg tgcgagtgtc ctggagccca gtccctggtg ccacccagta ccgcatcatt 1620

gtgcgcagca cccaggggt tgagcggacc ctggtgcttc ctgggagtca gacagcattc 1680

gacttggatg acgttcaggc tgggcttagc tacactgtgc gggtgtctgc tcgagtgggt 1740

ccccgtgagg gcagtgccag tgtcctcact gtccgccggg agccggaaac tccacttgct 1800

gttccagggc tgcgggttgt ggtgtcagat gcaacgcgag tgagggtggc ctggggaccc 1860

gtccctggag ccagtggatt tcggattagc tggagcacag gcagtggtcc ggagtccagc 1920

cagacactgc ccccagactc tactgccaca gacatcacag ggctgcagcc tggaaccacc 1980

taccaggtgg ctgtgtcggt actgcgaggc agagaggagg gccctgctgc agtcatcgtg 2040

gctcgaacgg acccactggg cccagtgagg acggtccatg tgactcaggc cagcagctca 2100

tctgtcacca ttacctggac cagggttcct ggcgccacag gatacagggt ttcctggcac 2160

tcagcccacg gcccagagaa atcccagttg gtttctgggg aggccacggt ggctgagctg 2220

gatggactgg agccagatac tgagtatacg gtgcatgtga gggcccatgt ggctggcgtg 2280

gatgggcccc ctgcctctgt ggttgtgagg actgcccctg agcctgtggg tcgtgtgtcg 2340

aggctgcaga tcctcaatgc ttccagcgac gttctacgga tcacctgggt aggggtcact 2400

ggagccacag cttacagact ggcctggggc cggagtgaag gcggccccat gaggcaccag 2460

atactcccag gaaacacaga ctctgcagag atccggggtc tcgaaggtgg agtcagctac 2520

tcagtgcgag tgactgcact tgtcggggac cgcgagggca cacctgtctc cattgttgtc 2580

actacgccgc ctgaggctcc gccagccctg ggacgcttc acgtggtgca gcgcggggag 2640

cactcgctga ggctgcgctg ggagccggtg cccagagcgc agggcttcct tctgcactgg 2700

caacctgagg gtggccagga acagtcccgg gtcctggggc ccgagctcag cagctatcac 2760

ctggacgggc tggagccagc gacacagtac cgcgtgaggc tgagtgtcct agggccagct 2820

ggagaagggc cctctgcaga ggtgactgcg cgcactgagt cacctcgtgt tccaagcatt 2880

gaactacgtg tggtggacac ctcgatcgac tcggtgactt tggcctggac tccagtgtcc 2940

agggcatcca gctacatcct atcctggcgg ccactcagag gccctggcca ggaagtgcct 3000

gggtccccgc agacacttcc agggatctca agctcccagc gggtgacagg gctagagcct 3060

ggcgtctctt acatcttctc cctgacgcct gtcctggatg gtgtgcgggg tcctgaggca 3120

tctgtcacac agacgccagt gtgcccccgt ggcctggcgg atgtggtgtt cctaccacat 3180

gccactcaag acaatgctca ccgtgcggag gctacgagga gggtcctgga gcgtctggtg 3240
ttggcacttg ggcctcttgg gccacaggca gttcaggttg gcctgctgtc ttacagtcat 3300
cggccctccc cactgttccc actgaatggc tcccatgacc ttggcattat cttgcaaagg 3360
atccgtgaca tgccctacat ggacccaagt gggaacaacc tgggcacagc cgtggtcaca 3420
gctcacagat acatgttggc accagatgct cctgggcgcc gccagcacgt accaggggtg 3480
atggttctgc tagtggatga acccttgaga ggtgacatat tcagccccat ccgtgaggcc 3540
caggcttctg ggcttaatgt ggtgatgttg ggaatggctg gagcggaccc agagcagctg 3600
cgtcgcttgg cgccgggtat ggactctgtc cagaccttct tcgccgtgga tgatgggcca 3660
agcctggacc aggcagtcag tggtctggcc acagccctgt gtcaggcatc cttcactact 3720
cagccccggc cagagccctg cccagtgtat tgtccaaagg gccagaaggg ggaacctgga 3780
gagatgggcc tgagaggaca agttgggcct cctggcgacc ctggcctccc gggcaggacc 3840
ggtgctcccg gcccccaggg gcccccctgga agtgccactg ccaagggcga gaggggcttc 3900
cctggagcag atgggcgtcc aggcagccct ggccgcgccg ggaatcctgg gacccctgga 3960
gcccctggcc taaagggctc tccagggttg cctggccctc gtggggaccc gggagagcga 4020
ggacctcgag gcccaaaggg ggagccgggg gctcccggac aagtcatcgg aggtgaagga 4080
cctgggcttc ctgggcggaa aggggaccct ggaccatcgg gcccccctgg acctcgtgga 4140
ccactggggg acccaggacc ccgtggcccc ccagggcttc ctggaacagc catgaagggt 4200
gacaaaggcg atcgtgggga gcggggtccc cctggaccag gtgaaggtgg cattgctcct 4260
ggggagcctg ggctgccggg tcttcccgga agccctggac cccaaggccc cgttggcccc 4320
cctggaaaga aaggagaaaa aggtgactct gaggatggag ctccaggcct cccaggacaa 4380
cctgggtctc cgggtgagca gggcccacgg ggacctcctg gagctattgg ccccaaaggt 4440
gaccggggct ttccagggcc cctgggtgag gctggagaga agggcgaacg tggaccccca 4500
ggcccagcgg gatcccgggg gctgccaggg gttgctggac gtcctggagc caagggtcct 4560
gaagggccac caggacccac tggccgccaa ggagagaagg gggagcctgg tcgccctggg 4620
gaccctgcag tggtgggacc tgctgttgct ggacccaaag gagaaaaggg agatgtgggg 4680
cccgctgggc ccagaggagc taccggagtc caaggggaac ggggcccacc cggcttggtt 4740
cttcctggag accctggccc caagggagac cctggagacc ggggtcccat tggccttact 4800
ggcagagcag gacccccagg tgactcaggg cctcctggag agaagggaga ccctgggcgg 4860
cctggccccc caggacctgt tggcccccga ggacgagatg gtgaagttgg agagaaaggt 4920
gacgagggtc ctccgggtga cccgggtttg cctggaaaag caggcgagcg tggccttcgg 4980
ggggcacctg gagttcgggg gcctgtgggt gaaaagggag accagggaga tcctggagag 5040

gatggacgaa atggcagccc tggatcatct ggacccaagg gtgaccgtgg ggagccgggt 5100
cccccaggac ccccgggacg gctggtagac acaggacctg gagccagaga gaagggagag 5160
cctggggacc gcggacaaga gggtcctcga gggcccaagg gtgatcctgg cctccctgga 5220
gcccctgggg aaaggggcat tgaagggttt cggggacccc caggcccaca gggggaccca 5280
ggtgtccgag gcccagcagg agaaaagggt gaccggggtc cccctgggct ggatggccgg 5340
agcggactgg atgggaaacc aggagccgct gggccctctg ggccgaatgg tgctgcaggc 5400
aaagctgggg acccagggag agacgggctt ccaggcctcc gtggagaaca gggcctccct 5460
ggcccctctg gtccccctgg attaccggga aagccaggcg aggatggcaa acctggcctg 5520
aatggaaaaa acggagaacc tggggaccct ggagaagacg ggaggaaggg agagaaagga 5580
gattcaggcg cctctgggag agaaggtcgt gatggcccca agggtgagcg tggagctcct 5640
ggtatccttg gaccccaggg gcctccaggc ctcccagggc cagtgggccc tcctggccag 5700
ggttttcctg gtgtcccagg aggcacgggc cccaagggtg accgtgggga gactggatcc 5760
aaaggggagc agggcctccc tggagagcgt ggcctgcgag gagagcctgg aagtgtgccg 5820
aatgtggatc ggttgctgga aactgctggc atcaaggcat ctgccctgcg ggagatcgtg 5880
gagacctggg atgagagctc tggtagcttc ctgcctgtgc ccgaacggcg tcgaggcccc 5940
aagggggact caggcgaaca gggcccccca ggcaaggagg gccccatcgg ctttcctgga 6000
gaacgcgggc tgaagggcga ccgtggagac cctggccctc aggggccacc tggtctggcc 6060
cttggggaga ggggcccccc cgggccttcc ggccttgccg gggagcctgg aaagcctggt 6120
attcccgggc tcccaggcag ggctgggggt gtgggagagg caggaaggcc aggagagagg 6180
ggagaacggg gagagaaagg agaacgtgga gaacagggca gagatggccc tcctggactc 6240
cctggaaccc ctgggccccc cggaccccct ggccccaagg tgtctgtgga tgagccaggt 6300
cctggactct ctggagaaca gggaccccct ggactcaagg gtgctaaggg ggagccgggc 6360
agcaatggtg accaaggtcc caaaggagac aggggtgtgc caggcatcaa aggagaccgg 6420
ggagagcctg gaccgagggg tcaggacggc aacccgggtc taccaggaga gcgtggtatg 6480
gctgggcctg aagggaagcc gggtctgcag ggtccaagag gcccccctgg cccagtgggt 6540
ggtcatggag accctggacc acctggtgcc ccgggtcttg ctggccctgc aggaccccaa 6600
ggaccttctg gcctgaaggg ggagcctgga gagacaggac ctccaggacg gggcctgact 6660
ggacctactg gagctgtggg acttcctgga ccccccggcc cttcaggcct tgtgggtcca 6720
caggggtctc caggtttgcc tggacaagtg gggagacag ggaagccggg agccccaggt 6780
cgagatggtg ccagtggaaa agatggagac agagggagcc ctggtgtgcc agggtcacca 6840
ggtctgcctg gccctgtcgg acctaaagga gaacctggcc ccacgggggc ccctggacag 6900
gctgtggtcg ggctccctgg agcaaaggga gagaagggag cccctggagg ccttgctgga 6960

gacctggtgg gtgagccggg agccaaaggt gaccgaggac tgccagggcc gcgaggcgag 7020

aagggtgaag ctggccgtgc aggggagccc ggagaccctg gggaagatgg tcagaaaggg 7080

gctccaggac ccaaaggttt caagggtgac ccaggagtcg ggtcccggg ctcccctggg 7140

cctcctggcc ctccaggtgt gaagggagat ctgggcctcc ctggcctgcc cggtgctcct 7200

ggtgttgttg ggttcccggg tcagacaggc cctcgaggag agatgggtca gccaggccct 7260

agtggagagc ggggtctggc aggcccccca gggagagaag gaatcccagg acccctgggg 7320

ccacctggac caccggggtc agtgggacca cctggggcct ctggactcaa aggagacaag 7380

ggagaccctg gagtagggct gcctgggccc cgaggcgagc gtggggagcc aggcatccgg 7440

ggtgaagatg gccgccccgg ccaggaggga ccccgaggac tcacggggcc ccctggcagc 7500

aggggagagc gtggggagaa gggtgatgtt gggagtgcag gactaaaggg tgacaaggga 7560

gactcagctg tgatcctggg gcctccaggc ccacggggtg ccaaggggga catgggtgaa 7620

cgagggcctc ggggcttgga tggtgacaaa ggacctcggg gagacaatgg ggaccctggt 7680

gacaagggca gcaagggaga gcctggtgac aagggctcag ccgggttgcc aggactgcgt 7740

ggactcctgg gaccccaggg tcaacctggt gcagcaggga tccctggtga cccgggatcc 7800

ccaggaaagg atggagtgcc tggtatccga ggagaaaaag gagatgttgg cttcatgggt 7860

ccccggggcc tcaagggtga acggggagtg aagggagcct gtggccttga tggagagaag 7920

ggagacaagg gagaagctgg tcccccaggc cgcccgggc tggcaggaca caaaggagag 7980

atgggggagc ctggtgtgcc gggccagtcg ggggcccctg gcaaggaggg cctgatcggt 8040

cccaagggtg accgaggctt tgacgggcag ccaggcccca agggtgacca gggcgagaaa 8100

ggggagcggg gaaccccagg aattgggggc ttcccaggcc ccagtggaaa tgatggctct 8160

gctggtcccc cagggccacc tggcagtgtt ggtcccagag gccccgaagg acttcagggc 8220

cagaagggtg agcgaggtcc ccccggagag agagtggtgg gggctcctgg ggtccctgga 8280

gctcctggcg agagagggga gcaggggcgg ccagggcctg ccggtcctcg aggcgagaag 8340

ggagaagctg cactgacgga ggatgacatc cggggctttg tgcgccaaga gatgagtcag 8400

cactgtgcct gccagggcca gttcatcgca tctggatcac gacccctccc tagttatgct 8460

gcagacactg ccggctccca gctccatgct gtgcctgtgc tccgcgtctc tcatgcagag 8520

gaggaagagc gggtaccccc tgaggatgat gagtactctg aatactccga gtattctgtg 8580

gaggagtacc aggaccctga agctccttgg gatagtgatg acccctgttc cctgccactg 8640

gatgagggct cctgcactgc ctacaccctg cgctggtacc atcgggctgt gacaggcagc 8700

acagaggcct gtcacccttt tgtctatggt ggctgtggag ggaatgccaa ccgttttggg 8760

acccgtgagg cctgcgagcg ccgctgccca ccccgggtgg tccagagcca ggggacaggt 8820

actgcccagg actga 8835

<210> 2
<211> 2944
<212> PRT
<213> Homo sapiens

<400> 2

Met Thr Leu Arg Leu Leu Val Ala Ala Leu Cys Ala Gly Ile Leu Ala
1 5 10 15

Glu Ala Pro Arg Val Arg Ala Gln His Arg Glu Arg Val Thr Cys Thr
20 25 30

Arg Leu Tyr Ala Ala Asp Ile Val Phe Leu Leu Asp Gly Ser Ser Ser
35 40 45

Ile Gly Arg Ser Asn Phe Arg Glu Val Arg Ser Phe Leu Glu Gly Leu
50 55 60

Val Leu Pro Phe Ser Gly Ala Ala Ser Ala Gln Gly Val Arg Phe Ala
65 70 75 80

Thr Val Gln Tyr Ser Asp Asp Pro Arg Thr Glu Phe Gly Leu Asp Ala
85 90 95

Leu Gly Ser Gly Gly Asp Val Ile Arg Ala Ile Arg Glu Leu Ser Tyr
100 105 110

Lys Gly Gly Asn Thr Arg Thr Gly Ala Ala Ile Leu His Val Ala Asp
115 120 125

His Val Phe Leu Pro Gln Leu Ala Arg Pro Gly Val Pro Lys Val Cys
130 135 140

Ile Leu Ile Thr Asp Gly Lys Ser Gln Asp Leu Val Asp Thr Ala Ala
145 150 155 160

Gln Arg Leu Lys Gly Gln Gly Val Lys Leu Phe Ala Val Gly Ile Lys
165 170 175

Asn Ala Asp Pro Glu Glu Leu Lys Arg Val Ala Ser Gln Pro Thr Ser
180 185 190

Asp Phe Phe Phe Phe Val Asn Asp Phe Ser Ile Leu Arg Thr Leu Leu
195 200 205

Pro Leu Val Ser Arg Arg Val Cys Thr Thr Ala Gly Gly Val Pro Val

210 215 220

Thr Arg Pro Pro Asp Asp Ser Thr Ser Ala Pro Arg Asp Leu Val Leu
225 230 235 240

Ser Glu Pro Ser Ser Gln Ser Leu Arg Val Gln Trp Thr Ala Ala Ser
245 250 255

Gly Pro Val Thr Gly Tyr Lys Val Gln Tyr Thr Pro Leu Thr Gly Leu
260 265 270

Gly Gln Pro Leu Pro Ser Glu Arg Gln Glu Val Asn Val Pro Ala Gly
275 280 285

Glu Thr Ser Val Arg Leu Arg Gly Leu Arg Pro Leu Thr Glu Tyr Gln
290 295 300

Val Thr Val Ile Ala Leu Tyr Ala Asn Ser Ile Gly Glu Ala Val Ser
305 310 315 320

Gly Thr Ala Arg Thr Thr Ala Leu Glu Gly Pro Glu Leu Thr Ile Gln
325 330 335

Asn Thr Thr Ala His Ser Leu Leu Val Ala Trp Arg Ser Val Pro Gly
340 345 350

Ala Thr Gly Tyr Arg Val Thr Trp Arg Val Leu Ser Gly Gly Pro Thr
355 360 365

Gln Gln Gln Glu Leu Gly Pro Gly Gln Gly Ser Val Leu Leu Arg Asp
370 375 380

Leu Glu Pro Gly Thr Asp Tyr Glu Val Thr Val Ser Thr Leu Phe Gly
385 390 395 400

Arg Ser Val Gly Pro Ala Thr Ser Leu Met Ala Arg Thr Asp Ala Ser
405 410 415

Val Glu Gln Thr Leu Arg Pro Val Ile Leu Gly Pro Thr Ser Ile Leu
420 425 430

Leu Ser Trp Asn Leu Val Pro Glu Ala Arg Gly Tyr Arg Leu Glu Trp
435 440 445

Arg Arg Glu Thr Gly Leu Glu Pro Pro Gln Lys Val Val Leu Pro Ser
450 455 460

Asp Val Thr Arg Tyr Gln Leu Asp Gly Leu Gln Pro Gly Thr Glu Tyr
465 470 475 480

Arg Leu Thr Leu Tyr Thr Leu Leu Glu Gly His Glu Val Ala Thr Pro
485 490 495

Ala Thr Val Val Pro Thr Gly Pro Glu Leu Pro Val Ser Pro Val Thr
500 505 510

Asp Leu Gln Ala Thr Glu Leu Pro Gly Gln Arg Val Arg Val Ser Trp
515 520 525

Ser Pro Val Pro Gly Ala Thr Gln Tyr Arg Ile Ile Val Arg Ser Thr
530 535 540

Gln Gly Val Glu Arg Thr Leu Val Leu Pro Gly Ser Gln Thr Ala Phe
545 550 555 560

Asp Leu Asp Asp Val Gln Ala Gly Leu Ser Tyr Thr Val Arg Val Ser
565 570 575

Ala Arg Val Gly Pro Arg Glu Gly Ser Ala Ser Val Leu Thr Val Arg
580 585 590

Arg Glu Pro Glu Thr Pro Leu Ala Val Pro Gly Leu Arg Val Val Val
595 600 605

Ser Asp Ala Thr Arg Val Arg Val Ala Trp Gly Pro Val Pro Gly Ala
610 615 620

Ser Gly Phe Arg Ile Ser Trp Ser Thr Gly Ser Gly Pro Glu Ser Ser
625 630 635 640

Gln Thr Leu Pro Pro Asp Ser Thr Ala Thr Asp Ile Thr Gly Leu Gln
645 650 655

Pro Gly Thr Thr Tyr Gln Val Ala Val Ser Val Leu Arg Gly Arg Glu
660 665 670

Glu Gly Pro Ala Ala Val Ile Val Ala Arg Thr Asp Pro Leu Gly Pro
675 680 685

Val Arg Thr Val His Val Thr Gln Ala Ser Ser Ser Ser Val Thr Ile
690 695 700

Thr Trp Thr Arg Val Pro Gly Ala Thr Gly Tyr Arg Val Ser Trp His
705 710 715 720

```
Ser Ala His Gly Pro Glu Lys Ser Gln Leu Val Ser Gly Glu Ala Thr
                725                 730                 735

Val Ala Glu Leu Asp Gly Leu Glu Pro Asp Thr Glu Tyr Thr Val His
            740                 745                 750

Val Arg Ala His Val Ala Gly Val Asp Gly Pro Pro Ala Ser Val Val
        755                 760                 765

Val Arg Thr Ala Pro Glu Pro Val Gly Arg Val Ser Arg Leu Gln Ile
    770                 775                 780

Leu Asn Ala Ser Ser Asp Val Leu Arg Ile Thr Trp Val Gly Val Thr
785                 790                 795                 800

Gly Ala Thr Ala Tyr Arg Leu Ala Trp Gly Arg Ser Glu Gly Gly Pro
                805                 810                 815

Met Arg His Gln Ile Leu Pro Gly Asn Thr Asp Ser Ala Glu Ile Arg
            820                 825                 830

Gly Leu Glu Gly Gly Val Ser Tyr Ser Val Arg Val Thr Ala Leu Val
        835                 840                 845

Gly Asp Arg Glu Gly Thr Pro Val Ser Ile Val Val Thr Thr Pro Pro
    850                 855                 860

Glu Ala Pro Pro Ala Leu Gly Thr Leu His Val Val Gln Arg Gly Glu
865                 870                 875                 880

His Ser Leu Arg Leu Arg Trp Glu Pro Val Pro Arg Ala Gln Gly Phe
                885                 890                 895

Leu Leu His Trp Gln Pro Glu Gly Gly Gln Glu Gln Ser Arg Val Leu
            900                 905                 910

Gly Pro Glu Leu Ser Ser Tyr His Leu Asp Gly Leu Glu Pro Ala Thr
        915                 920                 925

Gln Tyr Arg Val Arg Leu Ser Val Leu Gly Pro Ala Gly Glu Gly Pro
    930                 935                 940

Ser Ala Glu Val Thr Ala Arg Thr Glu Ser Pro Arg Val Pro Ser Ile
945                 950                 955                 960

Glu Leu Arg Val Val Asp Thr Ser Ile Asp Ser Val Thr Leu Ala Trp
                965                 970                 975
```

Thr Pro Val Ser Arg Ala Ser Ser Tyr Ile Leu Ser Trp Arg Pro Leu
980 985 990

Arg Gly Pro Gly Gln Glu Val Pro Gly Ser Pro Gln Thr Leu Pro Gly
995 1000 1005

Ile Ser Ser Ser Gln Arg Val Thr Gly Leu Glu Pro Gly Val Ser
1010 1015 1020

Tyr Ile Phe Ser Leu Thr Pro Val Leu Asp Gly Val Arg Gly Pro
1025 1030 1035

Glu Ala Ser Val Thr Gln Thr Pro Val Cys Pro Arg Gly Leu Ala
1040 1045 1050

Asp Val Val Phe Leu Pro His Ala Thr Gln Asp Asn Ala His Arg
1055 1060 1065

Ala Glu Ala Thr Arg Arg Val Leu Glu Arg Leu Val Leu Ala Leu
1070 1075 1080

Gly Pro Leu Gly Pro Gln Ala Val Gln Val Gly Leu Leu Ser Tyr
1085 1090 1095

Ser His Arg Pro Ser Pro Leu Phe Pro Leu Asn Gly Ser His Asp
1100 1105 1110

Leu Gly Ile Ile Leu Gln Arg Ile Arg Asp Met Pro Tyr Met Asp
1115 1120 1125

Pro Ser Gly Asn Asn Leu Gly Thr Ala Val Val Thr Ala His Arg
1130 1135 1140

Tyr Met Leu Ala Pro Asp Ala Pro Gly Arg Arg Gln His Val Pro
1145 1150 1155

Gly Val Met Val Leu Leu Val Asp Glu Pro Leu Arg Gly Asp Ile
1160 1165 1170

Phe Ser Pro Ile Arg Glu Ala Gln Ala Ser Gly Leu Asn Val Val
1175 1180 1185

Met Leu Gly Met Ala Gly Ala Asp Pro Glu Gln Leu Arg Arg Leu
1190 1195 1200

Ala Pro Gly Met Asp Ser Val Gln Thr Phe Phe Ala Val Asp Asp

```
        1205                1210                1215

Gly Pro Ser Leu Asp Gln Ala Val Ser Gly Leu Ala Thr Ala Leu
        1220                1225                1230

Cys Gln Ala Ser Phe Thr Thr Gln Pro Arg Pro Glu Pro Cys Pro
        1235                1240                1245

Val Tyr Cys Pro Lys Gly Gln Lys Gly Glu Pro Gly Glu Met Gly
        1250                1255                1260

Leu Arg Gly Gln Val Gly Pro Pro Gly Asp Pro Gly Leu Pro Gly
        1265                1270                1275

Arg Thr Gly Ala Pro Gly Pro Gln Gly Pro Pro Gly Ser Ala Thr
        1280                1285                1290

Ala Lys Gly Glu Arg Gly Phe Pro Gly Ala Asp Gly Arg Pro Gly
        1295                1300                1305

Ser Pro Gly Arg Ala Gly Asn Pro Gly Thr Pro Gly Ala Pro Gly
        1310                1315                1320

Leu Lys Gly Ser Pro Gly Leu Pro Gly Pro Arg Gly Asp Pro Gly
        1325                1330                1335

Glu Arg Gly Pro Arg Gly Pro Lys Gly Glu Pro Gly Ala Pro Gly
        1340                1345                1350

Gln Val Ile Gly Gly Glu Gly Pro Gly Leu Pro Gly Arg Lys Gly
        1355                1360                1365

Asp Pro Gly Pro Ser Gly Pro Pro Gly Pro Arg Gly Pro Leu Gly
        1370                1375                1380

Asp Pro Gly Pro Arg Gly Pro Pro Gly Leu Pro Gly Thr Ala Met
        1385                1390                1395

Lys Gly Asp Lys Gly Asp Arg Gly Glu Arg Gly Pro Pro Gly Pro
        1400                1405                1410

Gly Glu Gly Gly Ile Ala Pro Gly Glu Pro Gly Leu Pro Gly Leu
        1415                1420                1425

Pro Gly Ser Pro Gly Pro Gln Gly Pro Val Gly Pro Pro Gly Lys
        1430                1435                1440
```

```
Lys Gly  Glu Lys Gly Asp Ser  Glu Asp Gly Ala Pro  Gly Leu Pro
    1445                 1450                 1455

Gly Gln  Pro Gly Ser Pro Gly  Glu Gln Gly Pro Arg  Gly Pro Pro
    1460                 1465                 1470

Gly Ala  Ile Gly Pro Lys Gly  Asp Arg Gly Phe Pro  Gly Pro Leu
    1475                 1480                 1485

Gly Glu  Ala Gly Glu Lys Gly  Glu Arg Gly Pro Pro  Gly Pro Ala
    1490                 1495                 1500

Gly Ser  Arg Gly Leu Pro Gly  Val Ala Gly Arg Pro  Gly Ala Lys
    1505                 1510                 1515

Gly Pro  Glu Gly Pro Pro Gly  Pro Thr Gly Arg Gln  Gly Glu Lys
    1520                 1525                 1530

Gly Glu  Pro Gly Arg Pro Gly  Asp Pro Ala Val Val  Gly Pro Ala
    1535                 1540                 1545

Val Ala  Gly Pro Lys Gly Glu  Lys Gly Asp Val Gly  Pro Ala Gly
    1550                 1555                 1560

Pro Arg  Gly Ala Thr Gly Val  Gln Gly Glu Arg Gly  Pro Pro Gly
    1565                 1570                 1575

Leu Val  Leu Pro Gly Asp Pro  Gly Pro Lys Gly Asp  Pro Gly Asp
    1580                 1585                 1590

Arg Gly  Pro Ile Gly Leu Thr  Gly Arg Ala Gly Pro  Pro Gly Asp
    1595                 1600                 1605

Ser Gly  Pro Pro Gly Glu Lys  Gly Asp Pro Gly Arg  Pro Gly Pro
    1610                 1615                 1620

Pro Gly  Pro Val Gly Pro Arg  Gly Arg Asp Gly Glu  Val Gly Glu
    1625                 1630                 1635

Lys Gly  Asp Glu Gly Pro Pro  Gly Asp Pro Gly Leu  Pro Gly Lys
    1640                 1645                 1650

Ala Gly  Glu Arg Gly Leu Arg  Gly Ala Pro Gly Val  Arg Gly Pro
    1655                 1660                 1665

Val Gly  Glu Lys Gly Asp Gln  Gly Asp Pro Gly Glu  Asp Gly Arg
    1670                 1675                 1680
```

Asn Gly Ser Pro Gly Ser Ser Gly Pro Lys Gly Asp Arg Gly Glu
1685 1690 1695

Pro Gly Pro Pro Gly Pro Pro Gly Arg Leu Val Asp Thr Gly Pro
1700 1705 1710

Gly Ala Arg Glu Lys Gly Glu Pro Gly Asp Arg Gly Gln Glu Gly
1715 1720 1725

Pro Arg Gly Pro Lys Gly Asp Pro Gly Leu Pro Gly Ala Pro Gly
1730 1735 1740

Glu Arg Gly Ile Glu Gly Phe Arg Gly Pro Pro Gly Pro Gln Gly
1745 1750 1755

Asp Pro Gly Val Arg Gly Pro Ala Gly Glu Lys Gly Asp Arg Gly
1760 1765 1770

Pro Pro Gly Leu Asp Gly Arg Ser Gly Leu Asp Gly Lys Pro Gly
1775 1780 1785

Ala Ala Gly Pro Ser Gly Pro Asn Gly Ala Ala Gly Lys Ala Gly
1790 1795 1800

Asp Pro Gly Arg Asp Gly Leu Pro Gly Leu Arg Gly Glu Gln Gly
1805 1810 1815

Leu Pro Gly Pro Ser Gly Pro Pro Gly Leu Pro Gly Lys Pro Gly
1820 1825 1830

Glu Asp Gly Lys Pro Gly Leu Asn Gly Lys Asn Gly Glu Pro Gly
1835 1840 1845

Asp Pro Gly Glu Asp Gly Arg Lys Gly Glu Lys Gly Asp Ser Gly
1850 1855 1860

Ala Ser Gly Arg Glu Gly Arg Asp Gly Pro Lys Gly Glu Arg Gly
1865 1870 1875

Ala Pro Gly Ile Leu Gly Pro Gln Gly Pro Pro Gly Leu Pro Gly
1880 1885 1890

Pro Val Gly Pro Pro Gly Gln Gly Phe Pro Gly Val Pro Gly Gly
1895 1900 1905

Thr Gly Pro Lys Gly Asp Arg Gly Glu Thr Gly Ser Lys Gly Glu
1910 1915 1920

Gln Gly Leu Pro Gly Glu Arg Gly Leu Arg Gly Glu Pro Gly Ser
1925 1930 1935

Val Pro Asn Val Asp Arg Leu Leu Glu Thr Ala Gly Ile Lys Ala
1940 1945 1950

Ser Ala Leu Arg Glu Ile Val Glu Thr Trp Asp Glu Ser Ser Gly
1955 1960 1965

Ser Phe Leu Pro Val Pro Glu Arg Arg Arg Gly Pro Lys Gly Asp
1970 1975 1980

Ser Gly Glu Gln Gly Pro Pro Gly Lys Glu Gly Pro Ile Gly Phe
1985 1990 1995

Pro Gly Glu Arg Gly Leu Lys Gly Asp Arg Gly Asp Pro Gly Pro
2000 2005 2010

Gln Gly Pro Pro Gly Leu Ala Leu Gly Glu Arg Gly Pro Pro Gly
2015 2020 2025

Pro Ser Gly Leu Ala Gly Glu Pro Gly Lys Pro Gly Ile Pro Gly
2030 2035 2040

Leu Pro Gly Arg Ala Gly Gly Val Gly Glu Ala Gly Arg Pro Gly
2045 2050 2055

Glu Arg Gly Glu Arg Gly Glu Lys Gly Glu Arg Gly Glu Gln Gly
2060 2065 2070

Arg Asp Gly Pro Pro Gly Leu Pro Gly Thr Pro Gly Pro Pro Gly
2075 2080 2085

Pro Pro Gly Pro Lys Val Ser Val Asp Glu Pro Gly Pro Gly Leu
2090 2095 2100

Ser Gly Glu Gln Gly Pro Pro Gly Leu Lys Gly Ala Lys Gly Glu
2105 2110 2115

Pro Gly Ser Asn Gly Asp Gln Gly Pro Lys Gly Asp Arg Gly Val
2120 2125 2130

Pro Gly Ile Lys Gly Asp Arg Gly Glu Pro Gly Pro Arg Gly Gln
2135 2140 2145

Asp Gly Asn Pro Gly Leu Pro Gly Glu Arg Gly Met Ala Gly Pro

2150 2155 2160

Glu Gly Lys Pro Gly Leu Gln Gly Pro Arg Gly Pro Pro Gly Pro
2165 2170 2175

Val Gly Gly His Gly Asp Pro Gly Pro Pro Gly Ala Pro Gly Leu
2180 2185 2190

Ala Gly Pro Ala Gly Pro Gln Gly Pro Ser Gly Leu Lys Gly Glu
2195 2200 2205

Pro Gly Glu Thr Gly Pro Pro Gly Arg Gly Leu Thr Gly Pro Thr
2210 2215 2220

Gly Ala Val Gly Leu Pro Gly Pro Pro Gly Pro Ser Gly Leu Val
2225 2230 2235

Gly Pro Gln Gly Ser Pro Gly Leu Pro Gly Gln Val Gly Glu Thr
2240 2245 2250

Gly Lys Pro Gly Ala Pro Gly Arg Asp Gly Ala Ser Gly Lys Asp
2255 2260 2265

Gly Asp Arg Gly Ser Pro Gly Val Pro Gly Ser Pro Gly Leu Pro
2270 2275 2280

Gly Pro Val Gly Pro Lys Gly Glu Pro Gly Pro Thr Gly Ala Pro
2285 2290 2295

Gly Gln Ala Val Val Gly Leu Pro Gly Ala Lys Gly Glu Lys Gly
2300 2305 2310

Ala Pro Gly Gly Leu Ala Gly Asp Leu Val Gly Glu Pro Gly Ala
2315 2320 2325

Lys Gly Asp Arg Gly Leu Pro Gly Pro Arg Gly Glu Lys Gly Glu
2330 2335 2340

Ala Gly Arg Ala Gly Glu Pro Gly Asp Pro Gly Glu Asp Gly Gln
2345 2350 2355

Lys Gly Ala Pro Gly Pro Lys Gly Phe Lys Gly Asp Pro Gly Val
2360 2365 2370

Gly Val Pro Gly Ser Pro Gly Pro Pro Gly Pro Pro Gly Val Lys
2375 2380 2385

Gly Asp Leu Gly Leu Pro Gly Leu Pro Gly Ala Pro Gly Val Val
2390 2395 2400

Gly Phe Pro Gly Gln Thr Gly Pro Arg Gly Glu Met Gly Gln Pro
2405 2410 2415

Gly Pro Ser Gly Glu Arg Gly Leu Ala Gly Pro Pro Gly Arg Glu
2420 2425 2430

Gly Ile Pro Gly Pro Leu Gly Pro Pro Gly Pro Pro Gly Ser Val
2435 2440 2445

Gly Pro Pro Gly Ala Ser Gly Leu Lys Gly Asp Lys Gly Asp Pro
2450 2455 2460

Gly Val Gly Leu Pro Gly Pro Arg Gly Glu Arg Gly Glu Pro Gly
2465 2470 2475

Ile Arg Gly Glu Asp Gly Arg Pro Gly Gln Glu Gly Pro Arg Gly
2480 2485 2490

Leu Thr Gly Pro Pro Gly Ser Arg Gly Glu Arg Gly Glu Lys Gly
2495 2500 2505

Asp Val Gly Ser Ala Gly Leu Lys Gly Asp Lys Gly Asp Ser Ala
2510 2515 2520

Val Ile Leu Gly Pro Pro Gly Pro Arg Gly Ala Lys Gly Asp Met
2525 2530 2535

Gly Glu Arg Gly Pro Arg Gly Leu Asp Gly Asp Lys Gly Pro Arg
2540 2545 2550

Gly Asp Asn Gly Asp Pro Gly Asp Lys Gly Ser Lys Gly Glu Pro
2555 2560 2565

Gly Asp Lys Gly Ser Ala Gly Leu Pro Gly Leu Arg Gly Leu Leu
2570 2575 2580

Gly Pro Gln Gly Gln Pro Gly Ala Ala Gly Ile Pro Gly Asp Pro
2585 2590 2595

Gly Ser Pro Gly Lys Asp Gly Val Pro Gly Ile Arg Gly Glu Lys
2600 2605 2610

Gly Asp Val Gly Phe Met Gly Pro Arg Gly Leu Lys Gly Glu Arg
2615 2620 2625

```
Gly Val  Lys Gly Ala Cys Gly  Leu Asp Gly Glu Lys  Gly Asp Lys
    2630                 2635                 2640

Gly Glu  Ala Gly Pro Pro Gly  Arg Pro Gly Leu Ala  Gly His Lys
    2645                 2650                 2655

Gly Glu  Met Gly Glu Pro Gly  Val Pro Gly Gln Ser  Gly Ala Pro
    2660                 2665                 2670

Gly Lys  Glu Gly Leu Ile Gly  Pro Lys Gly Asp Arg  Gly Phe Asp
    2675                 2680                 2685

Gly Gln  Pro Gly Pro Lys Gly  Asp Gln Gly Glu Lys  Gly Glu Arg
    2690                 2695                 2700

Gly Thr  Pro Gly Ile Gly Gly  Phe Pro Gly Pro Ser  Gly Asn Asp
    2705                 2710                 2715

Gly Ser  Ala Gly Pro Pro Gly  Pro Pro Gly Ser Val  Gly Pro Arg
    2720                 2725                 2730

Gly Pro  Glu Gly Leu Gln Gly  Gln Lys Gly Glu Arg  Gly Pro Pro
    2735                 2740                 2745

Gly Glu  Arg Val Val Gly Ala  Pro Gly Val Pro Gly  Ala Pro Gly
    2750                 2755                 2760

Glu Arg  Gly Glu Gln Gly Arg  Pro Gly Pro Ala Gly  Pro Arg Gly
    2765                 2770                 2775

Glu Lys  Gly Glu Ala Ala Leu  Thr Glu Asp Asp Ile  Arg Gly Phe
    2780                 2785                 2790

Val Arg  Gln Glu Met Ser Gln  His Cys Ala Cys Gln  Gly Gln Phe
    2795                 2800                 2805

Ile Ala  Ser Gly Ser Arg Pro  Leu Pro Ser Tyr Ala  Ala Asp Thr
    2810                 2815                 2820

Ala Gly  Ser Gln Leu His Ala  Val Pro Val Leu Arg  Val Ser His
    2825                 2830                 2835

Ala Glu  Glu Glu Glu Arg Val  Pro Pro Glu Asp Asp  Glu Tyr Ser
    2840                 2845                 2850

Glu Tyr  Ser Glu Tyr Ser Val  Glu Glu Tyr Gln Asp  Pro Glu Ala
    2855                 2860                 2865
```

```
Pro Trp Asp Ser Asp Asp Pro Cys Ser Leu Pro Leu Asp Glu Gly
    2870                2875                2880

Ser Cys Thr Ala Tyr Thr Leu Arg Trp Tyr His Arg Ala Val Thr
    2885                2890                2895

Gly Ser Thr Glu Ala Cys His Pro Phe Val Tyr Gly Gly Cys Gly
    2900                2905                2910

Gly Asn Ala Asn Arg Phe Gly Thr Arg Glu Ala Cys Glu Arg Arg
    2915                2920                2925

Cys Pro Pro Arg Val Val Gln Ser Gln Gly Thr Gly Thr Ala Gln
    2930                2935                2940

Asp


<210>  3
<211>  20
<212>  DNA
<213>  Homo sapiens

<400>  3
accccacagt ggggccacta                                              20


<210>  4
<211>  20
<212>  DNA
<213>  Homo sapiens

<400>  4
gtcaccaatc ctgtccctag                                              20


<210>  5
<211>  20
<212>  DNA
<213>  Homo sapiens

<400>  5
ggggccacta gggacaggat                                              20
```